# EUROPEAN PATENT APPLICATION

(11) **EP 4 837 962 A1**
(43) Date of publication of application: **11.11.2026**
(21) Application number: 25382463.5
(22) Date of filing: 08.05.2025
(51) Int. Cl.: C12N 5/0775, A61K 35/28, C12N 5/077

(54) **METHOD OF OBTENTION OF AN IMMUNOMODULATORY PRODUCT COMPRISING A SECRETOME, AN EXOSOME OR MICROVESICLES, AND USES THEREOF**

(71) Applicant: Faminway S.L., 50002 Zaragoza (ES)
(72) Inventor: LAPUENTE FERNANDEZ, Juan Pedro, 50830 LAS LOMAS DEL GÁLLEGO (ES); LAPUENTE HERNANDEZ, Daniel, 50830 LAS LOMAS DEL GÁLLEGO (ES); LAPUENTE HERNANDEZ, BELEN, 50830 LAS LOMAS DEL GÁLLEGO (ES); GOMEZ CASTILLO, Sergio, 50830 LAS LOMAS DEL GÁLLEGO (ES); HERNANDEZ LOPEZ, Susana, 50830 LAS LOMAS DEL GÁLLEGO (ES); FORTES MADRIGAL, Antonio, 08940 Cornella de Llobregat (ES); GAVIER MORENO, Alejandra, 50002 Zaragoza (ES)
(74) Representative: Clarke, Modet y Cía., S.L.

(57) **Abstract**

The present invention is a method of obtention of an immunomodulatory product comprising a secretome, exosomes and/or microvesicles, the method comprising pre-conditioning of primary cells and Mesenchymal stem cells (MSC) or M2- or M1-activated macrophages obtained from a subject with a suitable culture medium each; co-culturing in a bioreactor the preconditioned cells in fresh medium, in which bioreactor each cell or cell type is physically separated from the other cell type, and incubating said co-culture under stirring for the obtention of an enriched medium; harvesting said enriched medium and filtering for the obtention of the immunomodulatory product. In a preferred aspect, said physical separation of cells is achieved by encapsulating the cells.

## Description

### FIELD OF INVENTION

The present invention belongs to the field of cellular biology, more specifically to the production of pharmaceutical preparations containing cellular components. The pharmaceutical preparations are allogeneic medicines applicable to immune-mediated, inflammatory or tumor diseases.

### BACKGROUND OF THE INVENTION

Cell co-culture is a laboratory technique used in cell biology and biomedicine that involves the simultaneous culture of two or more different cell types in the same controlled environment. This method allows cellular interactions to be studied more accurately and realistically, replicating the multicellular conditions found in the tissues and organs of living organisms.

Co-cultures facilitate the investigation of complex cellular dynamics, such as intercellular communication, the influence of the cellular microenvironment, and coordinated responses to external stimuli. This technique is fundamental to understanding key biological processes, such as tissue development, regeneration, disease progression, and response to drug treatments. To date, above all, it is fundamental to obtain possible drugs based on the use of a complete secretome or some of its fractions to treat specific diseases. Two-dimensional (2D) or three-dimensional (3D) co-cultures can be performed under static or dynamic conditions, depending on the specific objectives of the study. Interactions among cells in a co-culture can be direct, when cells are in physical contact, or indirect, when cells are separated but share the same culture medium, allowing the exchange of soluble factors. Each of these approaches has its own advantages and limitations, and the choice of a co-culture system depends largely on the specific research question and the type of cells that are involved.

2D co-cultures represent one of the most widely used techniques in cell research due to their simplicity and ability to provide detailed information on intercellular interactions in a controlled environment. Different cell types are grown, and the cells adhere to the substrate surface and grow in a single flat layer. The most common methods for establishing 2D co-cultures include sequential seeding, co-seeding or physical separation with Transwell Inserts.

Transwell Inserts are easy to use, cost-effective, and allow direct observation. However, this technique has limitations such as offering a simplified representation of the living tissue, limited spatial interaction since cellular interactions are restricted to two dimensions, or challenges in nutrient and growth factor diffusion, leading to often inaccurate modeling of in vivo conditions. Besides, 2D cellular interactions may affect the physiological relevance of the results with potential impacts on cell function, intracellular organization, secretion and signaling.

The third dimension bridges the gap between cell culture and live tissue. 3D co-cultures represent a significant advance in cellular research, providing a more physiologically relevant environment to study cellular interactions and tissue architecture. In 3D co-cultures, cells are grown in an environment that supports growth and organization in three dimensions using natural or synthetic extracellular matrices, multicellular spheroids, organoids, or 3D bioprinting systems. The resulting co-cultures offer more realistic and predictive models of diseases, regenerative medicine, drug development and toxicology. 3D co-cultures can replicate the structure, function and cellular conditions of living tissues in a realistic microenvironment, or creating gradients for nutrients, oxygen and growth factors, closely mimicking *in vivo* conditions (Ravi M et al. (2015). 3D cell culture systems: Advantages and applications. Journal of Cellular Physiology, 230(1), 16-26). On the other hand, the limitations are their technical complexity, high cost and reproducibility challenges.

Static co-cultures involve the growth of different cell types together in a controlled environment without continuous streams of culture medium. This approach allows cell interactions to be studied under simpler, more controlled conditions. The cells are seeded and grow either on a 2D flat surface or in a 3D matrix without continuous movement of the culture medium. The most common configurations used in this type of co-culture are 2D monolayers and 3D arrays (Rajan N & D'Souza LS (2019). Co-culture of different cell types for tissue engineering applications: Protocols and challenges. Journal of Tissue Engineering and Regenerative Medicine, 13(9), 1687-1703).

The transwell systems described in the art do not allow precise and dynamic control of the microenvironment. Methods in these systems are also entirely manual. A medium renewal is always needed and in transwell systems is typically periodic rather than continuous, affecting the stability of culture conditions. Transwell inserts have limited cell volume and density capacity, restricting the amount of produced secretome. The batch-to-batch variability and manual handling may introduce inconsistencies in secretome production and, therefore, the scalability is more complicated and less efficient compared to bioreactors. Automation is virtually impossible to be implemented in Transwell systems, affecting process reproducibility and efficiency. Cells are more exposed to the external environment - this can affect their viability and endurance, wherein the lack of additional protection against external factors may compromise a continuous and sustained production of secretomes from the culture. Cellular interactions in Transwell systems are more constrained and the ability to create and maintain specific local microenvironments is reduced, potentially limiting the diversity, complexity and quality of the secretome - and this is so because they are unable to create dynamic cultures.

Dynamic co-cultures represent an advanced technique in biomedical research that more accurately simulates the physiological conditions of living tissues by using continuous streams of culture medium. Dynamic bioreactors or microfluidic devices ensure a constant supply of nutrients and oxygen while removing waste products. The co-cultures achieve an enhanced physiological relevance, allowing complex modelling that enables the study of cellular interactions and biological responses within an environment that closely mimics the conditions of tissues and organs.

A cell bioreactor allows precise control over parameters such as pH, temperature, dissolved oxygen and nutrient concentration, optimizing conditions for a secretome production. It enables a continuous perfusion of culture medium and removal of waste products, thereby improving long-term cell viability and functionality. This capability supports the development of a fully automated industrial process, helping for a consistent quality minimizing human intervention. This facilitates manufacturing in a GMP environment. However, establishing an indirect contact cell co-culture in a bioreactor involves several technical and biological challenges that have hindered the effective implementation of this technology in the art.

Controlling culture conditions and culture media optimization for different cell types is complex, as well as achieving effective cellular interactions. Intercellular signaling involving cytokines, chemokines, and exosomes must be adequate to induce the desired responses in the different cell types. Incomplete understanding of these complex interactions represents a significant obstacle. In addition, the signals may change over time depending on the state of the cells. The bioreactor operational design requires a physical separation of the cells while allowing for effective exchange of signals and nutrients. The scalability and reproducibility of co-cultures from laboratory to industrial-scale production in bioreactors is also challenging, depending on differences in fluid dynamics and mass transfer. Cross-contamination among different cell types, and genetic and phenotypic cell stability over time is critical, and the costs for developing bioreactors for co-culture, culture methods and control protocols including specialized personnel operating these systems is high. The combination of all these factors turns out that establishing an indirect contact cell co-culture in a bioreactor is a complex task that has not yet been successfully achieved.

Biological immunomodulating products typically comprise five categories: glucocorticoids; systemic compounds with immunomodulatory activity; cytotoxic agents and antimetabolites; monoclonal antibodies and receptor antagonists that block the action of pro-inflammatory cytokines; and immunoglobulins used as modulators of the immune response.

Cellular interactions can be mediated by cytokines and chemokines, exosomes and microvesicles by cell surface receptors and by direct cell-cell interactions.

Amongst the immunomodulatory agents, M2-macrophages inhibit inflammation producing polyamines to induce proliferation or proline to induce collagen production, whereas M1-macrophages promote inflammation by the production of nitric oxide or reactive oxygen intermediates. The role of M1-macrophages is to secrete pro-inflammatory cytokines and chemokines to present antigens and thus participate in the positive immune response and function as an immune monitor. The main pro-inflammatory cytokines are IL-6, IL-12 and TNF-alpha. The three types identified of M2-macrophages, M2a, M2b, and M2c, mainly secrete Arginase-I, IL-10 and TGF-β and other anti-inflammatory cytokines, with the function of reducing inflammation and contributing to tumor growth and Immunosuppressive function, playing an important role in wound healing and tissue repair.

To date, drugs comprising immunomodulatory products have been manufactured using a variety of strategies, such as tissue engineering and organ-on-a-chip, cell co-cultures, or advanced bioprocessors, or application of Artificial Intelligence and Machine Learning (Organ-on-a-chip: recent breakthroughs and future prospects. BioMed Eng OnLine 19, 9; Kaushik G & Wesa A (2021); Ackermann M et al. (2024) 3D co-culture platform reveals insights into patient autologous immune cell-tumor interaction and immune modulation in vitro Journal for ImmunoTherapy of Cancer 2021;9).

An emerging area in the development of these drugs is the use of cellular crosstalk, referring to the interaction and communication between different cell types to influence their behaviors and functions. Cellular crosstalk involves a series of biochemical and physical signals that allow cells to coordinate their activities (Abbas AK et al. (2020). Basic Immunology: Functions and Disorders of the Immune System (6th ed.). Elsevier). As new technologies emerge and our understanding of cellular interactions deepens, this approach is expected to lead to the development of more effective and personalized treatments for a wide range of diseases. Achieving this potential requires industrial scalability that is currently absent, as the methods discussed above for producing cell co-cultures through indirect contact are highly manual and offer limited scalability. This limitation is made evident in the absence of phase III clinical trials involving exosomes produced by co-culture of two cell types. The legal requirement for GMP certification, which requires a high level of automation and industrial scalability, remains a significant barrier for advancing these therapies to the clinical stage.

Also used in the present invention, cell microencapsulation is a technique consisting of enclosing cells within a semi-permeable membrane providing protection and control over their microenvironment for growth function in bioreactors. The materials suitable for encapsulation are typically natural polymers Microencapsulation can be performed by extrusion techniques, which involve passing a flow of cells in a polymer solution through a fine needle forming droplets that jellify upon contact with a cross-linking solution, coacervation, emulsion in which the cells are mixed with a polymer solution and dispersed in a continuous phase forming microdroplets that are subsequently cross-linked, or electrospray employing an electric field to produce fine droplets of polymer solution containing cells *(*Cell Microencapsulation: Methods and Protocols, Humana Press, Wang H et al., 2024)

Microcapsules are a type of microparticle, generally ranged from 0.01 to 1 µm, consisting of a core and a protective shell that contains bioactive substances. They offer many benefits, including extended drug release, increased bioavailability of therapeutic molecules, and decreased drug toxicity. Cell encapsulation in hydrogel allows to produce 3D cell cultures at microscale.

It remains challenging to establish an automated cell co-culture system in a bioreactor involving indirect contact between two or more cell types. This challenge is particularly evident in efforts to produce immunomodulatory products and drugs based on a secretome, microvesicles and/or exosomes, due to the high complexity of the physical requirements involved.

The problem of the art can be considered as an obtention method of complex specific immunomodulatory products more representative of the live environment of the cells and natural interaction among cell types, complying with GMP requirements, capable of being escalated.

### DESCRIPTION OF THE INVENTION

### BRIEF DESCRIPTION OF THE INVENTION

The present invention relates to a method for mass producing allogeneic drugs applicable to different immune-mediated inflammatory or tumor diseases, based on a technique of co-culture of two different cell lines: one immune cell, typically a macrophage, MSCs and/or monocytes, and the other being a primary and/or mesenchymal cell belonging to a specific tissue, which acts as a signaling cell for the immune cell. As immunomodulatory products, the present invention obtains a complete secretome of the co-culture, the exosome, microvesicles, or the exosomes and microvesicles together purified from the secretome.

The present invention is a method of obtention of an immunomodulatory product comprising a secretome, exosomes and/or microvesicles, the method comprising pre-conditioning of primary cells and Mesenchymal stem cells (MSCs) or M2- or M1-activated macrophages obtained from a subject with a suitable culture medium each; co-culturing in a bioreactor the preconditioned cells in fresh medium, in which bioreactor each cell or cell type is physically separated from the other cell type, and incubating said co-culture under stirring for the obtention of an enriched medium; harvesting said enriched medium and filtering for the obtention of the immunomodulatory product.

In particular, the present invention is a method of obtention of an immunomodulatory product comprising a secretome, exosomes and/or microvesicles, the method comprising pre-conditioning of primary cells and Mesenchymal stem cells (MSCs) or M2- or M1-activated macrophages obtained from a subject with a suitable culture medium each; co-culturing the preconditioned cells in a bioreactor comprising the steps of introducing the culture medium for primary cells fresh in a bioreactor; introducing the pre-conditioned primary cells, wherein the amount of primary cells is of maximal 20% w/v of the previously added culture medium; incubating at a temperature of 35-40°C, pH between 7.2 - 7.4, during 12 - 36 h.; then introducing the culture medium of said MSCs or M2- or M1-activated macrophages fresh in the bioreactor, wherein the ratio of primary cell culture medium to MSC- or activated macrophage culture medium is between 1:2 and 2:1; introducing the pre-conditioned MSCs or activated macrophages, wherein the amount of MSCs or activated macrophages is of maximal 20% w/v of the culture medium of said cells that has been added in the bioreactor, to form a co-culture with the primary cells, in which each cell type is physically separated from other cell type; and incubating said co-culture under stirring at a temperature of 35 - 40°C and pH between 7.2 - 7.4 under hypoxia for the obtention of an enriched medium; harvesting said enriched medium and filtering by complex pulsed Tangential Flow Filtration (TFF), for the obtention of the immunomodulatory product.

The physical separation of cell types can be achieved by any mechanical means inside the bioreactor, preferably grouping the cell types in different compartments within the bioreactor or also preferably using microcapsules encapsulating each cell, isolating them from any other direct cell contact.

All temperatures and time referred to in the present application are subjected to an experimental error of ±0.5.

The membrane pore size of the TFF and the filtering procedure will determine the final obtention of a purified secretome, exosomes, microvesicles, or exosomes and microvesicles together.

In the present application, the secretome is defined comprising a complex mixture of bioactive molecules including soluble factors such as cytokines, chemokines, and growth factors, as well as other soluble proteins and extracellular vesicles, such as apoptotic bodies, microvesicles and exosomes that may contribute to immunomodulation, tissue regeneration, and therapeutic effects through autocrine and paracrine intercellular signaling. A preferred aspect of the invention is the purified secretome obtained by the method of the present invention.

In the present application, exosomes are defined as nanosized extracellular vesicles ranging from 30 to 150 nm in diameter, comprising proteins, lipids, nucleic acids and other bioactive molecules that contribute to intercellular communication and therapeutic effects. Exosomes are primarily identified by a high surface expression of at least three tetraspanines, CD9, CD63, and CD81, which distinguishes them from microvesicles that are typically larger but could eventually overlap in size. A preferred aspect of the invention is the exosomes obtained by the method of the present invention.

In the present application, microvesicles are defined as membrane-derived vesicles typically ranging from over 150 to 1000 nm in diameter, comprising bioactive molecules contributing to immune modulation and tissue repair. A preferred aspect of the invention is the microvesicles obtained by the method of the present invention.

Still another aspect is an immunomodulatory product obtained by the method of the invention comprising both microvesicles and exosomes formed by the indirect interaction of primary cells with MSCs or M2- or M1-activated macrophages.

Another aspect is directed to a pharmaceutical composition comprising the secretome, the exosomes and/or the microvesicles obtained by the method described above, and a pharmaceutically acceptable excipient.

Another aspect is directed to the pharmaceutical composition comprising any of the secretome, the exosome and/or the microvesicles indicated above, for use as a medicament.

Another aspect is directed to the pharmaceutical composition comprising any of the secretome, the exosome and/or the microvesicles indicated above, for use in the prophylaxis or treatment of type-1 diabetes, urinary incontinence, tendinosis, chronic neuropathic pain, pseudoarthrosis or rib or vertebral fractures, antibiotic-resistant infections, lung storm cytokines associated with CAR-**T,** acute pancreatitis and/or sepsis, Acute Myocardial Infarction (AMI), stroke, baldness, dermal aging, Benign Prostate Hyperplasia (BPH) or solid or liquid tumors (colon cancer, prostate cancer, etc.)

The immunostimulatory products obtained by the method of the invention at conditions similar to the physiological environment are expected more complex with respect to the art and certainly having a better therapeutical effect.

Still a further aspect is a method of preventing or treating type-1 diabetes, urinary incontinence, tendinosis, chronic neuropathic pain, pseudoarthrosis or rib or vertebral fractures, antibiotic-resistant infections, lung storm cytokines associated with CAR-T, acute pancreatitis and/or sepsis, Acute Myocardial Infarction (AMI), stroke, baldness, dermal aging, Benign Prostate Hyperplasia (BPH) or solid or liquid tumors (colon cancer, prostate cancer, etc.), the method comprising administering to a patient an effective amount of any of the secretome, the exosome and/or the microvesicles obtained by the method of the present invention.

In different preferred aspects, type-1 diabetes and pancreatic cancer are treated by exosomes obtained by the method of the invention, wherein the primary cells are human umbilical cord stem cells; Urinary incontinence is treated by exosomes obtained by the process of the invention, wherein the primary cells are myocytes co-cultured with M2 macrophages; Tendinosis is treated by exosomes obtained by the process, wherein the primary cells are tenocytes co-cultured with M2 macrophages; Chronic neuropathic pain is treated by exosomes obtained by the process, wherein the primary cells are Schwann cells co-cultured with M2 macrophages; Pseudoarthrosis or rib or vertebral fractures are treated by exosomes obtained by the process, wherein the primary cells are osteoblasts co-cultured with M2 macrophages; Antibiotic-resistant infections are treated by exosomes obtained by the process, wherein the primary cells are CD8/CD4 lymphocytes and/or M1 macrophages cocultured with bacteria; Lung storm cytokines associated with CAR-T, acute pancreatitis and/or sepsis are treated by exosomes obtained by the process, wherein the primary cells are adult mesenchymal stem cells co-cultured with M2 macrophages; AMI is treated by exosomes obtained by the process, wherein the primary cells are cardiomyocytes co-cultured with M2 macrophages; Stroke is treated by exosomes obtained by the process, wherein the primary cells are glial cells co-cultured with M2 macrophages; Baldness is treated by exosomes obtained by the process, wherein the primary cells are fibroblasts co-cultured with M2 macrophages; Dermal aging is treated by exosomes obtained by the process, wherein the primary cells are porcine umbilical cord mesenchymal cells co-cultured with M2 macrophages; BPH is treated by exosomes obtained by the process, wherein the primary cells are prostate epithelial and stromal cells co-cultured with M2 macrophages; and tumors are treated by exosomes obtained by the process, wherein the primary cells are tumor cells tumor-specific co-cultured with M1 macrophages.

The pharmaceutical composition can be administrated in different forms and routes. Secretome and exosome-based products and drugs have several routes and methods of administration, depending on the condition treated, the desired effects, and the biological properties of the components. Some of the suitable forms and routes are intravenous (IV) wherein exosomes or the secretome are administered directly into the bloodstream; IV administration provides rapid and uniform systemic biodistribution allowing components to reach multiple tissues efficiently. The limitations are the risk of immune response, potential for systemic side effects. By intraperitoneal (IP) administration, the exosomes are injected into the peritoneal cavity, typically in animal models and in specific studies; IP administration provides a systemic distribution with less risk of immune effects. The limitation is that this route is less common in humans and can cause peritoneal irritation. An intranasal administration has the advantage of being a noninvasive option with potential direct access of the exosomes to the brain and Central Nervous System (CNS), although this route has limited efficacy in some patients due to anatomical variations or nasal inflammation. By inhalation, the exosomes are absorbed into the respiratory epithelium, with the advantage of being a good option for lung diseases, since the drug is distributed in the lungs and allows systemic absorption; however, this is a device-dependent technique and requires stability of the exosomes in aerosol form. The intramuscular (IM) and intradermal Route has the advantage of providing for a sustained release over time, which is useful for local applications or when prolonged absorption is sought. However, it has little bioavailability and may cause discomfort or pain at the injection site; Subcutaneous (SC) injection has the advantage of a sustained release and gradual absorption into the blood stream, typically useful for chronic applications; however, it involves a slow absorption and potential local irritation. The intrathecal route is an injection into the subarachnoid space, near the spinal cord, and has the advantage of allowing direct access to the CNS, which is ideal for treatments of neurological diseases but is also an invasive technique with associated risks, such as infection or nerve damage. Intra-articular administration is indicated for inflammatory or degenerative joint diseases and provides localized effects, reducing the need to administer high systemic doses; however, it is an invasive technique with risk of infection or inflammation in the joint. Oral administration is non-invasive and convenient, but it challenges with the stability of exosomes due to the acidic environment of the stomach and digestive enzymes, requiring specific protection technology. Topical or transdermal applications are convenient and noninvasive and allow for sustained release, although it is limited to local applications and requires technology to enhance skin penetration. Ophthalmic administration directly into the eyes is ideal for ocular diseases, needing appropriate formulation to maintain the stability of the exosomes in an ocular environment avoiding irritation). Controlled release implants in form of biodegradable devices or implanted materials can be loaded with exosomes or secretome and applied at the treatment site, allowing prolonged and controlled release of the treatment, minimizing the need for multiple applications).

### BRIEF DESCRIPTION OF THE FIGURES

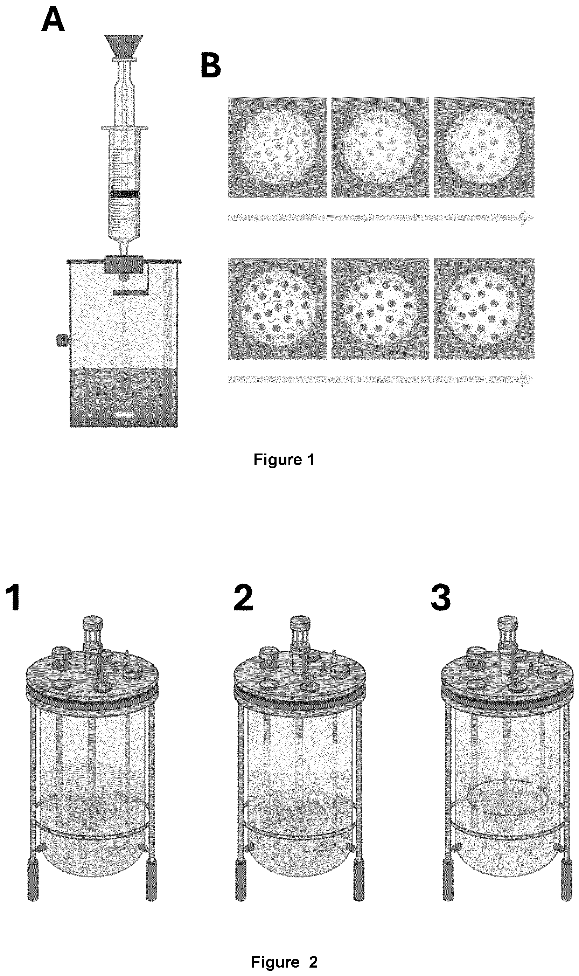

**Figure 1** shows the microencapsulation of cells in alginate capsules. (A) Encapsulation Mechanism: an alginate solution, a mix of alginate, porogen, and the cells to be encapsulated, is loaded into a syringe. The reaction vessel contains the polymerization solution with divalent cations. After establishing the necessary parameters, microdroplets of the alginate solution are produced, which gel upon contact with the polymerization solution. (B) Gelation Process: the carboxyl groups of the G-blocks in the alginate form ionic bonds with the divalent cations, creating a hydrogel that encapsulates the cells (ASCs or M2a macrophages) within the capsule.
**Figure 2****.** Indirect contact coculture in a Bioreactor: (1) Microencapsulated human Adipose-Derived Stem Cells (ASCs) are added first, along with their corresponding culture medium. (2) After 24 hours, microencapsulated M2a macrophages are added, also with their corresponding medium. Once both cell types are in the bioreactor, dynamic culture takes place (3) with stirring at a speed sufficient to ensure homogeneous mixing of the microcapsules without causing mechanical breakage.

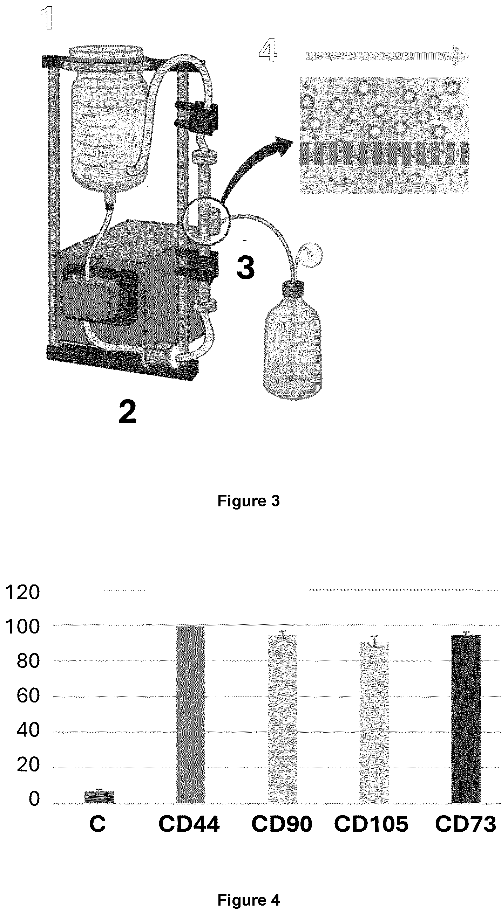

**Figure 3****.** Tangential Flow Filtration (TFF) of the Secretome: (1) The conditioned secretome from the bioreactor is transferred to the tank of the tangential flow filtration system. (2) The recirculation pump directs the secretome to the hollow fiber membrane. Applying pressure causes that part of the flow stream passes through a membrane as permeate. This technique provides continuous removal of accumulated materials, minimizing membrane fouling. The TFF equipment consists of a sterile glass tank that is filled through one of the ports on its top cover. The recirculation pump allows the secretome to be conducted from the tank to the filter unit, and from there, the retained volume (containing larger molecules above the MWCO or the pore size of the filter) is recirculated back to the tank as a retentate, while the filtered volume (containing smaller molecules below the MWCO or the pore size of the filter) forms the permeate. In addition to removing the smallest particles, the sample can be concentrated by directing the retentate to an auxiliary reservoir instead of returning it back to the tank. (3) At this point in the circuit, there is an inlet port for PBS, which is used for buffer exchange. By adjusting the addition of PBS, the concentration of secretome or extracellular vesicles in the final product can also be controlled. (4) Inside the hollow fiber membranes, the retentate of extracellular vesicles (large spheres) continue to flow and return to the tank, while permeate of smaller particles in terms of molecular weight or size (small spheres) pass through the filter and are separated from the extracellular vesicles. Meanwhile, the culture medium is gradually replaced with PBS.
**Figure 4****.** Percentage of positive cells for each antibody for the final product of the exosomes of Example 3 by ASCs immunostaining. The figure represents the flow cytometry characterization of ASCs, showing the percentage of cells expressing specific surface markers. ASCs were positive for key mesenchymal markers CD44, CD73, CD90 and CD105.

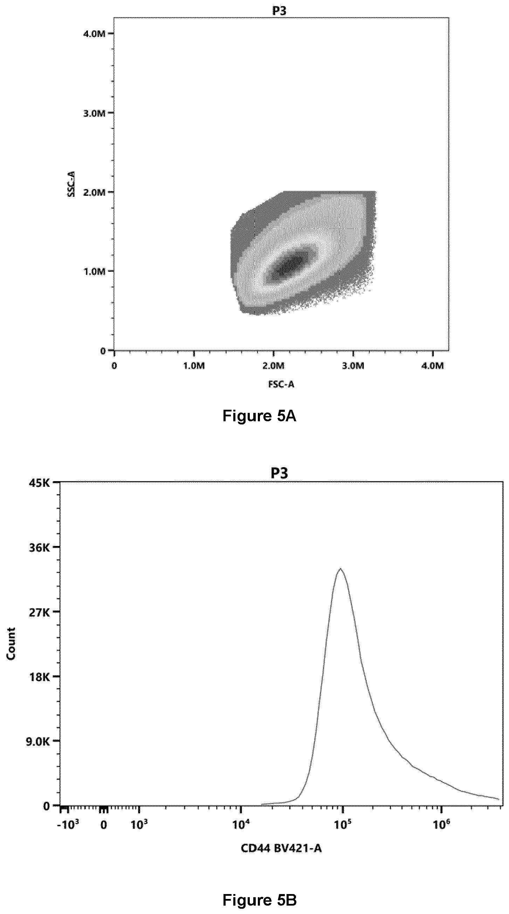

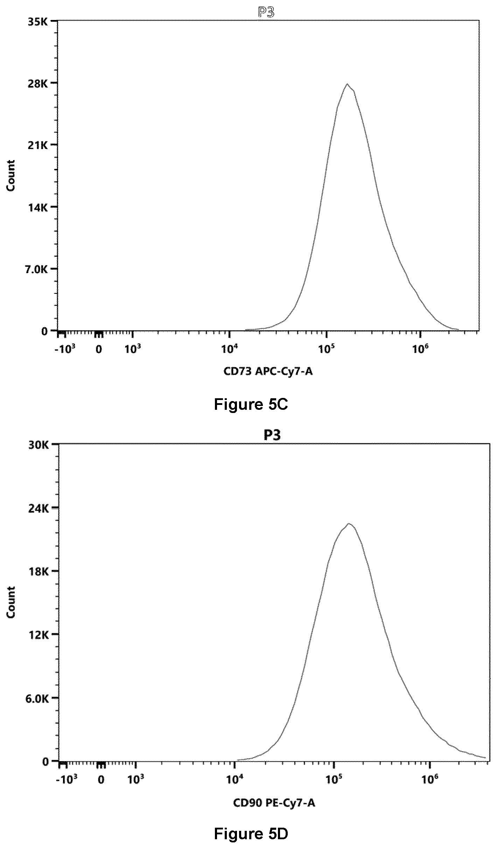

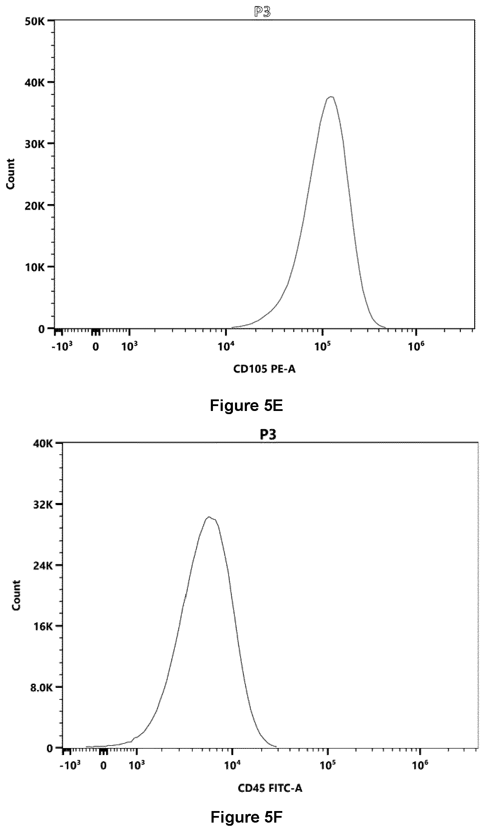

**Figure 5** shows: A) A representative flow cytometry dot plot showing side and forward scatter of human Adipose-Derived Stem Cells (ASCs) used in the manufacturing of the exosomes, and their membrane marker staining profile, with B) CD44, C) CD73, D) CD90 and E) CD105 as positive markers, and F) CD45, a hematopoietic marker, as a negative marker, confirming their identity as mesenchymal stem cells.

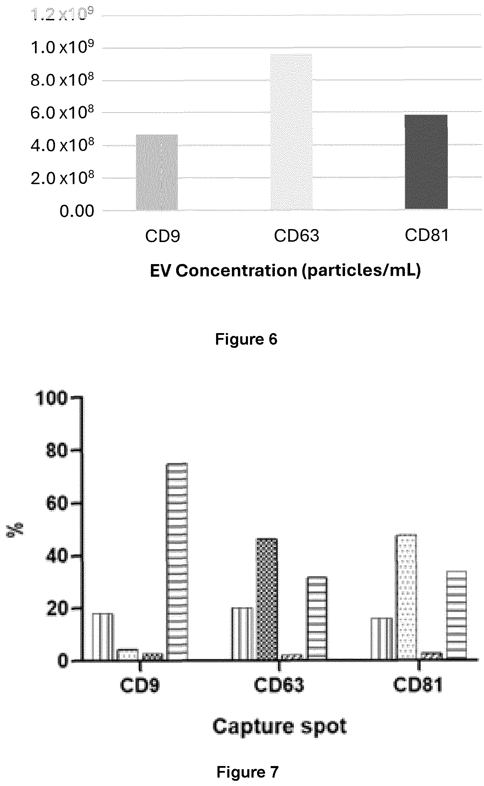

**Figure 6****.** Concentration of particles captured by CD9, CD63 and CD81 antibodies in exosomes obtained by the process of Example 3, indicating that CD63-positive exosomes are predominant.
**Figure 7****.** Colocalization of tetraspanins in exosomes. It represents single-positive exosomes (vertical lines), triple-positive exosomes (horizontal lines), and double-positive exosomes, classified as follows: Capture spot tetraspanin with CD9 (diagonal lines), capture spot tetraspanin with CD63 (small dots), and capture spot tetraspanin with CD81 (big dots). CD63- and CD81-positive exosomes show identical colocalization patterns, unlikely CD9-positive exosomes, which are less frequent and tend to be triple-positive for all the tetraspanins analyzed. This pattern may indicate that the exosomes possess immunomodulatory properties, as they may be multivesicular body-derived.

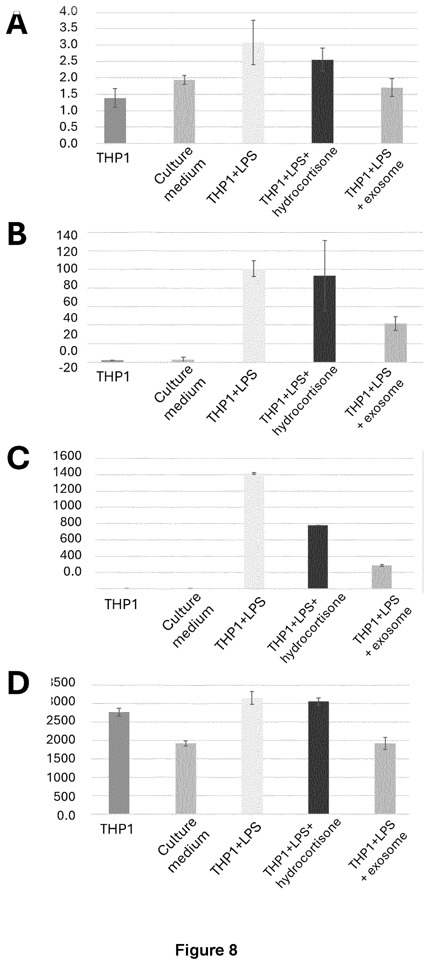

**Figure 8****.** Potency test of the exosome recovered in Example 3. (A) IFN-γ; (B): IL-1β; (C): TNF-α; (D): IL-R6α. A reduction of 55.3 % in IL-1β (B) and more than 70% reduction in TNF-α (C) was observed. This reduction indicates that the exosomes exhibit a clear anti-inflammatory effect.

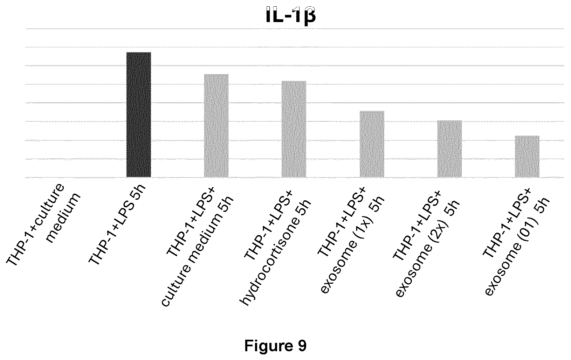

**Figure 9****.** Potency test with different concentrations of exosomes obtained in Example 3. The results demonstrate an anti-inflammatory response in a dose-dependent manner.

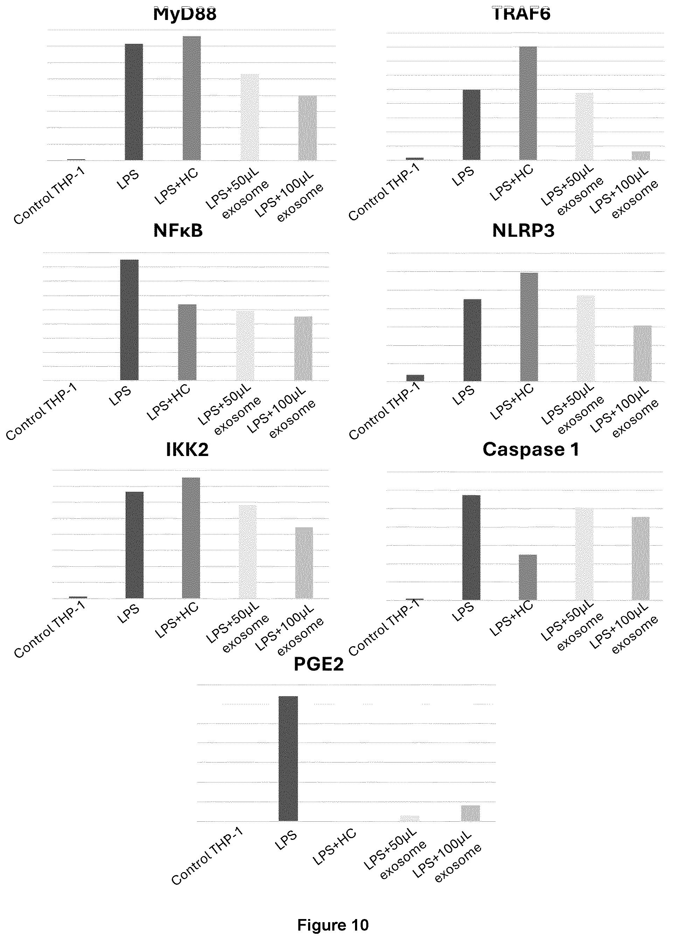

**Figure 10****.** Mechanism of Action (MoA) of the exosomes obtained by the method of the invention in an inflammatory macrophage model (THP-1, TLR-4 with LPS): The results show a reduction in the relative expression of several proteins in the TLR-4 activation pathway.

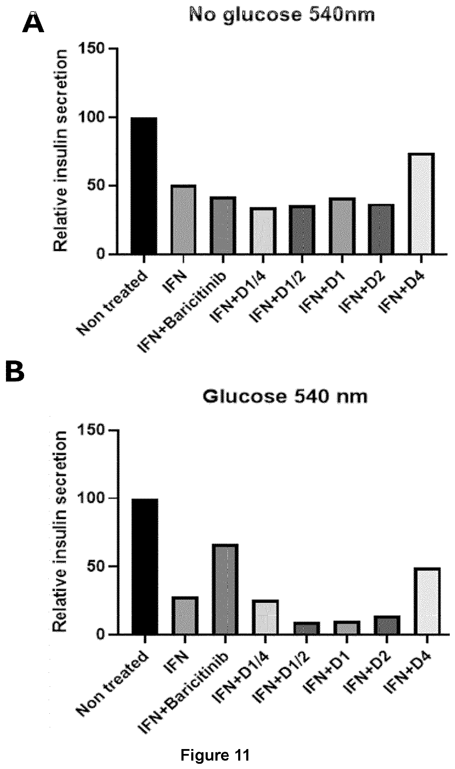

**Figure 11** shows that the Glucose-Stimulated Insulin Secretion (GSIS) experiment in Example 2 demonstrates the ability of the exosomes to recover the insulin production of pancreatic beta cells in presence (B) or absence (A) of glucose. The exosomes recover part of the insulin production of pancreatic beta cells at 4x dose, better than baricitinib in the non-stimulated condition and in a similar way in the stimulated one.

### DETAILED DESCRIPTION OF THE INVENTION

The invention is directed to a process of obtention of an immunomodulatory product comprising the following steps:
a) Pre-conditioning of primary cells, obtained from a subject in a suitable culture medium.
b) Pre-conditioning of Mesenchymal stem cells MSCs, or M2- or M1-activated macrophages (derived from Peripheral Blood Mononuclear cells PBMCs) obtained from a subject, in a suitable culture medium.

In the present application, "primary cells" are cells that have been obtained from tissue of the human or animal body, e.g. epithelial cells, fibroblasts, keratinocytes, melanocytes, endothelial cells, muscle cells, hematopoietic cells, or mesenchymal stem cells, by biopsy, surgery, or autopsy; or can also be acquired from certified cell banks, where they are isolated, expanded, and quality-controlled for research or clinical use. Most primary cells are terminally differentiated cells with a highly specific type and high level of function.

The composition of the pre-conditioning medium for primary cells can be adjusted to promote specific functional stimulation, as required. All cell types involved in the co-culture, including primary cells, MSCs and M2- or M1-activated macrophages, undergo pre-conditioning prior to co-culturing.

In the present application, a pre-conditioning step is defined as the process of culturing cells in monoculture to stabilize, acclimate, and optimize their viability and responsiveness before encapsulation. The pre-conditioning media for each cell type include cell-specific growth factors, supplements, and culture media tailored to support the cell growth and enhance overall cell function. For all cell types, pre-conditioning is preferably maintained for 3 to 7 days or until approximately 90% confluency is reached. Incubation parameters are set at 35 - 40°C, preferably 37°C, pH 7.2 - 7.4, 95% humidity, and 5% CO₂. Cultures are maintained under hypoxia and static conditions.

The culture medium used for each cell type along the method of the invention can be any serum-free medium described in the art that is absent of phenol red and gentamicin. Animal-derived components are likely replaced with synthetic or recombinant alternatives. For macrophages, MSCs, and dendritic cells, it is preferable to use a culture medium specifically developed for these cell types. For most primary cells, a more general medium, such as high-glucose DMEM, can be utilized. As known by the expert, the primary components of these culture media include glucose, in addition to inorganic salts, amino acids and, to a lesser extent, vitamins. Regardless of the culture medium selected, it must be supplemented according to the specific requirements of the cell type, including the addition of relevant growth factors or cytokines to support cell growth and enhance overall cell function. Essential supplements for all cell types include penicillin, streptomycin, and L-glutamine. In a preferred aspect, Mesenchymal Stem Cells (MSCs) are cultured in StemPro MSC SFM medium without phenol red, supplemented with 1% L-glutamine, 1% penicillin-streptomycin, and optionally 0.5% amphotericin B. In another preferred aspect, macrophages previously differentiated from monocytes are cultured in CTS AIM-V medium without phenol red, supplemented with 1% L-glutamine, 1% penicillin-streptomycin, 10 ng/mL M-CSF, and optionally 0.5% amphotericin B. In another preferred aspect, primary cells, excluding dendritic cells, are cultured in high-glucose DMEM medium without phenol red, supplemented with 5% human platelet lysate, 1% L-glutamine, 1% penicillin-streptomycin, 1% cell-specific growth supplement (Cardiac myocyte growth supplement, CMGS, for cardiac myocytes; Chondrocyte growth supplement, CGS, for chondrocytes; Fibroblast growth supplement, FGS, for fibroblasts; Keratinocyte growth supplement, KGS, for keratinocytes; Osteoblast growth supplement, OGS, for osteoblasts; Schwann cell growth supplement, SCGS, for Schwann cells; Skeletal muscle cell growth supplement, skMCGS, for skeletal muscle cells; and Tenocyte growth supplement, TGS, for tenocytes), and optionally 0.5% amphotericin B. In another preferred aspect, dendritic cells previously differentiated from monocytes are cultured in CTS AIM-V medium without phenol red, supplemented with 5% human platelet lysate, 1% L-glutamine, 1% penicillin-streptomycin, 5 ng/mL TNF-α, 5 ng/mL IL-1β, 150 ng/mL IL-6 and 1 µg/mL prostaglandin E2 (PGE2), and optionally 0.5% amphotericin B.

Pre-conditioning the cells prior to the co-culture with indirect contact is essential for several reasons: Adapting and stabilizing the cells to the culture conditions before facing the co-culture environment, which may have different nutrients, growth factors, and molecules secreted by other cells; Increasing cell viability and reducing variability in co-culture; Inducing the expression of specific cellular response factors such as cytokines, growth factors, or adhesion molecules; Optimizing the cellular response in co-culture to produce exosomes, cytokines and other specific factors that will be necessary for intercellular communication in co-culture; and allowing a closer control over the initial experimental conditions, since it is easier to identify which changes are due to interactions in co-culture and which are inherent to the cells.
c) co-culturing the cells of step a) and b) separated in a bioreactor, the co-culture comprising the steps of:
i. introduction of the fresh culture medium specific for the primary cells into a bioreactor, preferably filling around 60% of its working volume. The working volume typically corresponds to 20 - 40% of the total nominal capacity, more preferably around 30%;
ii. introduction of the pre-conditioned primary cells obtained in step a) in the bioreactor, wherein the amount of primary cells is of maximal 20% w/v or less of the culture medium added in step i., preferably 5% w/v;
iii. incubation at 35 - 40°C, preferably 37°C, at pH between 7.2 - 7.4 and during 12 - 36 hours, more preferably for 24 hours. In another preferred aspect, the incubation is conducted under hypoxic and/or static conditions.

In the present application, hypoxia is defined as an environment with less than 21% of dissolved O₂ (DO).

In the present application, static culture is defined as a culture with no movement or stirring, and with no aeration or an aeration rate of 0.05 - 0.2 vvm. "Vvm" stands for "1 volume of air per volume of medium per minute" and serves to describe the rate of aeration in bioreactors particularly in the context of microbial fermentation and cell culture. In a preferred aspect, the primary cells are statically cultured with an aeration rate of 0.1 vvm, with 5% CO₂, and 20% DO. In opposite, stirring involves any active movement of the culture medium sufficient to prevent sedimentation of cells, yet avoiding creating shear forces that would damage said cells.
iv. introduction of the fresh culture medium specific for MXCs or activated macrophages into the bioreactor, preferably filling the remaining 40% of its working volume. The ratio of primary cell culture medium to MSC- or activated macrophage culture medium is between 1:2 and 2:1, preferably between 1:1 and 2:1, even more preferably 1.5:1.
v. Introduction of the pre-conditioned MSCs or activated macrophages obtained in step b) into the bioreactor, wherein the amount of MSCs or activated macrophages is of maximal 20% w/v of the culture medium added in step iv., preferably 5% w/v.

It is essential that the co-culture is performed with physical separation between the cell types, avoiding direct cell-to-cell contact. This separation can be achieved by whichever mechanical means. A preferable aspect is forming microcapsules independently encapsulating the primary cells, MSCs and M2- or M1-activated macrophages obtained in steps a) and b), respectively, in natural polymers such as alginate, chitosan, gelatin, collagen, agarose or carrageenan, or synthetic polymers such as Poly(Lactic-co-Glycolic) Acid (PLGA), Poly-L-Lysine (PLL) or Poly(ethylene glycol) (PEG), more preferably in alginate and/or alginate combined with PLL or chitosan polymers. Alginate and alginate/PLL or alginate/chitosan polymers are preferred for having high biocompatibility, permeability, ability to undergo in situ ionic crosslinking and tunable properties. Since alginate lacks cell adhesion motifs, a preferred aspect of the invention adds extracellular matrix components such as collagen to enhance cell attachment and viability, particularly in cases where anchorage-dependent cells require support for optimal behavior within the microenvironment. By encapsulating each cell and cell type, the cells still get indirect contact through the micropores of the microcapsules. The microcapsules of the present invention have enough thickness, resistance and flexibility to withstand the collisions among them without breakage, thereby maintaining the size of the micropores in such a way that the exit of micromolecules for intercellular communication is allowed, inducing the cell types in co-culture to specific secretions based on this external stimulus.

Microencapsulation of cells to maintain separation during co-culture can limit the diffusion of secreted extracellular microvesicles, particularly larger microvesicles, due to the semi-permeable nature of the capsule membrane. With the aim of overcoming this limitation and ensure efficient release of all secreted products into the culture medium, a preferred aspect includes the incorporation of porogens, such as polyethylene glycol (PEG) or polyethylene oxide (PEO), into the alginate solution intended for encapsulation. These porogens improve membrane permeability, facilitating the passage of extracellular vesicles and soluble factors without retention inside the microcapsule.

In another aspect of the invention, the introduction of culture medium and corresponding cells are switched. The culture medium and pre-conditioned MSCs or activated macrophages of step b) can be incorporated in the bioreactor before the culture medium and the pre-conditioned primary cells of step a).
vi. Incubation of the co-culture of step v. under stirring, preferably at a speed of 15 - 30 rpm and even more preferably at an aeration rate of 0.05 - 0.2 vvm, at a temperature of 35 - 40°C, more preferably 37°C, pH between 7.2 - 7.4, and under hypoxia, for the obtention of an enriched medium containing a secretome, exosomes and microvesicles of the cells. In a preferred aspect, said incubation is performed for 10 - 20 days, preferably for 15 - 18 days.
d) Harvesting the enriched culture medium, preferably between 40 - 60% of volume of the total medium and also preferably every 3 days of culturing, for the obtention of a secretome including exosomes and microvesicles. The harvested volume is preferably replaced in the bioreactor with fresh medium, maintaining the ratio between the primary cell culture medium to MSC- or activated macrophage culture medium of the starting co-culture.
e) Filtration of said enriched culture medium for the obtention of the secretome, exosomes and microvesicles as immunomodulatory products, preferably using a complex Tangential Flow Filtration (TFF)-pulsed process.

For the obtention of the whole retentate comprising a purified secretome, exosome and microvesicles, the filtering is preferably performed with a 10 - 50 kDa membrane in a first concentration of the secretome, removing small impurities, such as soluble proteins and metabolites.

For the obtention of a combination of exosomes and microvesicles, the filtering is preferably performed with a 100 - 300 kDa membrane, withdrawing the complete permeate soluble part.

For the obtention of exosomes and microvesicles separately, a first filtration is preferably performed with a 100 - 300 kDa membrane, followed by a second filtration with a 0,1 - 0,2 µm pore size membrane. The exosomes will flow to the permeate, whereas the microvesicles will be retained in the retentate of this second filtration. To further isolate larger microvesicles, another aspect of the invention comprises a third filtration using a membrane with a higher pore size, preferably from 0,3 to 1 µm. Increasing the pore size allows for the isolation of progressively larger microvesicles in the retentate.

In the present application, the term "purifying" refers to the removal of the smallest soluble proteins, metabolites, and other contaminants primarily derived from the cell culture medium, from the first secretome obtained when harvesting medium, resulting in a concentrated secretome.

Another aspect of the invention is the retrieval of the encapsulated cells. This would involve the dissolution of the microcapsules, in case that this was the method used for separating the cells. The capsules can be dissolved using a sodium citrate or EDTA depolymerization solution. Following dissolution, the reaction is neutralized with PBS and the obtained solution be filtered to remove residual polymer fragments. The recovered free cells can then be pelleted by centrifugation and subsequently expanded, pre-conditioned, or used in further processes or analyses, e.g. for an evaluation of their post-culture viability and phenotype.

In the case that the secretome, exosome and/or microvesicles constituting the immunomodulatory product of the invention are intended for an immunomodulatory drug suitable for therapy, the following steps need to be added:
f) viral clearance of the obtained purified product;
g) optionally, filling of unidose packs in sterile apyrogenic containers under sterile conditions;
   and
h) optionally, lyophilization of the unidose packs of step f).

In the present application, "sterile conditions" are defined as an environment that is completely free of living microorganisms, such as bacteria, viruses, fungi, and spores, making use of sterile or disinfected materials under a laminar flow hood or biosafety cabinet.

In the present application, an immunomodulator or immunomodulatory product or drug is defined as a naturally or synthetically derived agent that can modulate, either stimulates or suppresses, both innate and adaptive immune systems.

In an aspect of the invention, the viral clearance of step f) comprises a methylene blue photochemical treatment. More preferably, the sample is mixed with methylene blue and irradiated for 15 - 20 minutes using visible light at approximately 630 nm. The obtained product consists of a virus-free solution of concentrated secretome, exosomes and/or microvesicles in a fresh buffer, preferably PBS o physiological serum.

In another aspect, the method includes a step g), where the secretome, exosomes and/or microvesicles are filled in sterile apyrogenic containers under sterile conditions. Preferably, the solution product is aliquoted into vials, filling each vial with 2 mL of product, previously mixed with 0,01 g of mannitol per mL of product to prevent damage during freezing. The vials may be stored at -80°C for 48 hours.

In another aspect, the method includes a final step h), where the vials are lyophilized after steps f) or g). Any lyophilization protocol known in the art can be followed. The final product is a dry, powdered secretome, exosomes and/or microvesicles concentrate that can be stored in the refrigerator at temperatures between 2 and 8°C or also frozen at -20°C. For reconstitution, it will be necessary to add the same volume of diluent, typically PBS, to obtain a 1x concentration.

The method of the present invention offers advantages over other indirect contact co-cultures described in the art.

The process described herein provides a precise control of the microenvironment. The mechanical separation of the cells enables high cell density in a small volume, allowing the creation of specific local microenvironments that influences the composition and complexity of the secretome. The obtained secretome is more diverse and functional since bioreactors facilitate more realistic interactions between the different cell types. The cells stirred in the bioreactor represent an improved model of physiological conditions for a co-culture with no direct interaction of cell types, wherein cells typically exhibit longer survival and functionality, extending the capacity of secretome production. The bioreactor offers a more reproducible and controlled environment, reducing variability between experiments and batches, and are easily scalable allowing large-scale production for therapeutic and industrial applications.

The proposed technology lies in the possibility to industrially manufacturing on a large scale and in a fully automated way of several lines of immunomodulatory biological drug types, based on the establishment of the co-culture of several different cell types in the same recipient with no direct contact among them. The method allows the establishment of a fully automated downstream line with the capability to generate the final product.

### EXAMPLES

### Example 1. Production of secretome by co-culture of ASCs and M2a macrophages

This example shows the production of secretome via indirect contact co-culture of Adipose-Derived Stem Cells (ASCs), a subset of Mesenchymal Stem Cells (MSCs) and M2a macrophages in a bioreactor.

### 1.1 Obtention of M2-PBMCs

The macrophages are obtained by differentiating monocytes into M0 macrophages with PMA, followed by polarization into M1-type macrophages with IFN-γ and/or IL-1β plus M-CSF, or into M2-type with IL-4 and IL-10 plus M-CSF.

If the objective is to treat an inflammatory disease with the aim of immunoregulating the inflammation, monocytes will be differentiated into M2a macrophages. M2a macrophages are able to secret high levels of anti-inflammatory cytokines, mainly IL-10. However, if the objective is to tag a cell that must be destroyed, as is the case with tumors, monocytes will be differentiated into M1 macrophages, the pro-inflammatory subtype whose secretome includes cytokines such as TNF-α, IL-1β and IL-6. The cell type that enters into indirect coculture with M2a or M1 macrophages determines the therapeutic target of the final product.

Work was always carried out under sterile conditions and manipulations were carried out in a laminar flow hood (Bio II Advance Plus 4, Telstar). Leukocyte-enriched blood bags (buffy coat) were obtained by leukapheresis. The material was transferred into 50 mL Falcon tubes. A density gradient centrifugation with Ficoll-Plaque Premium 1.073 (Cytiva) was performed, with a ratio of 15 mL Ficoll per 25 mL blood. Tubes were centrifuged for 30 min at 400 x g, without brake. A clean band corresponding to PBMCs (Peripheral Blood Mononuclear Cells) at interphase was collected as soon as possible to avoid precipitation into the erythrocyte phase.

Subsequent washes were performed using Phosphate Saline Buffer (PBS, Gibco). Before each centrifugation, tubes were filled with PBS to a final volume of 50 mL:
- Centrifugation 1200 rpm, 10 minutes: The supernatant was discarded, and the pellet was resuspended in a small residual volume of supernatant (< 5 mL) before adding PBS.
- Centrifugation 1000 rpm, 10 minutes: The supernatant was discarded, and the pellet was resuspended in 10 mL of red blood lysis buffer. PBS was not added at this step. It is important to avoid this lysis step earlier, as plasma present in the sample inactivate the lysis buffer.

- Centrifugation 800 rpm, 10 minutes: The supernatant was discarded, and the pellet was resuspended in a small residual volume of supernatant (< 5 mL) before adding PBS. If erythrocyte debris remained, a second lysis step was performed following the same procedure as in step 2.
- Centrifugation 800 rpm, 10 minutes: The supernatant is discarded.

All centrifugations were performed using a SL1R Plus centrifuge (Thermo Scientific). The final cell pellet was resuspended in 40 mL of monocyte culture medium, consisting of CTS AIM-V medium without phenol red (Gibco), supplemented with 1% L-glutamine (Gibco), 1% penicillin/streptomycin (Gibco), and optionally 0.5% amphotericin B (Corning). The cells were counted using the TC20 Automated Cell Counter (Bio Rad), and seeded in T175 flasks (Corning) at a density of 1 million cells/cm², in a final volume of 20 - 25 mL (culture medium + cell suspension). The cells were incubated overnight at 37°C in a 5% CO₂ atmosphere using the MCO-170AICUVH CO₂ incubator (PHCbi). After incubation, the medium enriched with lymphocytes and other non-adherent PBMCs was removed.

To induce monocyte differentiation into M0 macrophages, the adherent monocytic layer was first washed with 10 - 15 mL of PBS. Then, monocyte culture medium supplemented with 20 ng/mL PMA (Phorbol Myristate Acetate, Sigma Aldrich), was added to the flask. The cells were incubated at 37°C and 5% CO₂ for 48 hours. After incubation, the PMA-containing medium was removed, and the resulting M0 macrophages were washed carefully with PBS to remove residual PMA. Fresh monocyte culture medium was added, and the cells were incubated at 37°C and 5% CO₂ for 24 hours. The cultured cells were washed twice with 10 - 15 mL of PBS, and monocyte culture medium supplemented with 10 ng/mL M-CSF (PeproTech) was added. The cells were incubated at 37°C, 5% CO₂ for 4 days, with medium replacement every 24 hours.

On day 4, the corresponding cytokines were added:
a) Differentiation to M2a macrophages: IL-4 (20 ng/mL) (PeproTech) and IL-10 (20 ng/mL) (PeproTech), together with M-CSF, were added to the culture medium containing the M0 macrophages, and the cells were incubated for 24 hours at 37°C with 5% CO₂. Once polarization into M2a macrophages was complete, the medium was discarded, and the cells were washed twice with PBS.
b) Differentiation to M1 macrophages: IL-1β (10 ng/mL) (PeproTech) and/or IFN-γ (10 ng/mL) (PeproTech), together with M-CSF, were added to the culture medium containing the M0 macrophages, and the cells were incubated for 7 days at 37°C with 5% CO₂. Once polarization into M1 macrophages was complete, the medium was discarded, and the cells were washed twice with PBS.

### 1.2 Pre-conditioning cultures

The activated macrophages obtained as described in section 1.1 were cultured in macrophage culture medium, consisting of CTS AIM-V medium without phenol red, supplemented with 1% L-glutamine, 1% penicillin-streptomycin, 10 ng/mL M-CSF, and optionally 0.5% amphotericin B.

Separately, ASCs were obtained from the applicant's master cell bank. Prior to seeding, the flask was pre-treated with fibronectin (Innoprot) to achieve a final surface concentration of 2 µg/cm²: A coating solution of fibronectin in distilled water was added to the flask, followed by gentle 8-shaped movements to ensure even distribution. The flask was incubated for 1 hour at 37°C, 5% CO₂. The coating solution was removed, and the culture surface was washed twice with PBS. Following the coating procedure, ASCs were cultured at a density of 5000 cells/cm² in StemPro MSC SFM medium without phenol red (Gibco), supplemented with 1% L-glutamine, 1% penicillin-streptomycin, and optionally 0.5% amphotericin B.

For both macrophages and ASCs, culture maintenance, incubation conditions, and subculturing were performed according to the same procedure. Cells were incubated under static and hypoxic conditions at 37°C, pH 7.2, 95% humidity, and 5% CO₂. Medium changes were performed every 2 - 3 days until cultures reached approximately 90% confluency, typically within 3 - 7 days. Upon reaching confluency, cells were subcultured as follows: 1 mL of accutase (Corning) per 10 mL of medium was added slowly to the culture surface, followed by gentle figure-8 movements to ensure even distribution. Flasks were incubated for 5 minutes at 37°C and 5% CO₂. To facilitate detachment, the flask was tapped firmly on the sides and checked under microscope to ensure no cells remained adhered. A volume of culture medium equivalent to 4 times the volume of accutase was then added to neutralize the enzymatic action. The cell suspension was transferred to a 50 mL tube (Falcon) and centrifuged at 1200 rpm for 7 minutes. The resulting pellet was resuspended in 1 mL of the respective culture medium, and viable cells were counted.

### 1.3 Cell encapsulation

To encapsulate the cells, it was used the Encapsulator B-395 Pro (Buchi). This equipment works through a vibrating system that atomizes the alginate solution and cells in the form of small drops, which when they come into contact with the gelation solution form the microcapsules.

In first place, an alginate solution at 2% (w/v) was prepared dissolving alginic acid sodium salt from brown algae (Sigma Aldrich) in glucose solution (Gibco) at room temperature, and then filtered through a 0.2 µm filter. Then, a 1% (w/v) polyethylene oxide (Thermo Scientific Chemicals) was slowly added to the alginate solution, until the volumes of the two solutions reached a 1:1 ratio. The mixture was stirred at 40°C at 400 rpm for 4 to 6 hours.

The polymerization solution was prepared by mixing 100 mM CaCl₂ (Corning), 85 mM glucose (Gibco) and 10 mM HEPES (Gibco). The reaction vessel of the encapsulator was filled with 225 mL of the polymerization solution.

A 10 mL cell suspension was dispersed into 50 mL of the previously prepared alginate/PEO solution to achieve a final cell density between 2×10⁶ and 1×10⁷ cells/mL. This mixture was then loaded into a syringe and connected to the encapsulator.

Before initiating microcapsule formation, the conditions of the encapsulator were set within the following rates: nozzle diameter 150 µm; flow rate 3 mL/min; vibration frequency starting at 1200 Hz and then adjusting until a clear microcapsule chain is obtained; and voltage starting at 500 V, increasing in increments of 100 V, until achieving a circular dispersion of the microcapsule stream 3 to 10 cm after the electrode.

After configuring the encapsulator, the microcapsule formation began, with gelation occurring when the cellular solution came into contact with the polymerization solution, at 5 minutes after droplet immersion). During this process, the polymerization solution is stirred to avoid aggregates. Once all microcapsules were formed, the stirrer stopped, and the polymerization solution was drained, maintaining a small volume so that the microcapsules remain embedded in liquid. Afterwards, the reaction vessel was filled with lactated Ringer's solution (Thermo Scientific Chemicals), used as a washing buffer to rinse the microcapsules for 1 to 5 minutes before discarding it. The microcapsules were then transferred to the bead collection bottle and subsequently to the bioreactor, where they were cultured with the appropriate culture medium depending on the encapsulated cell type.

### 1.4 Co-culture in bioreactor

M2a macrophages are co-cultured with ASCs in the F0-Baby bioreactor (Bionet), which has a total nominal capacity of approximately 3 L and a working capacity of 2 L.

Firstly, the ASC culture medium (composition defined in section 1.2) was introduced into the bioreactor, filling approximately 60% of its working volume, which in this case is 1.2 L. The encapsulated ASCs were introduced and left for 24 hours in static culture, at 37°C, aeration rate of 0.1 vvm, 5% CO₂, 20% DO, and pH between 7.2 - 7.4. After 24 hours, the remaining working volume (0.8 L) was filled with macrophage culture medium (composition defined in section 1.2), and then the encapsulated M2a macrophages were introduced in the bioreactor. At this point, dynamic culture stirring began at a speed of 20 rpm, while maintaining the same conditions of 37°C, aeration rate of 0.1 vvm, 5% CO₂, 20% DO, and pH between 7.2 - 7.4. Every 3 days of co-culture (on days 3, 6, 9 and 12 of co-culture), 50% of the culture medium was removed using a 100 µm mesh filter, collecting the medium while the encapsulated cells remained in the bioreactor. Fresh medium was then added, maintaining the ratio of ASC culture medium to macrophage culture medium at 1.5:1. The co-culture ended after 15 days. On day 15, all the media were collected. In total, 6 L of secretome were obtained for each 15-day co-culture in a bioreactor. After the co-culture, all the media were collected and cryopreserved at -80°C for subsequent processing and lyophilization.

### 1.5 TFF filtration

To remove small impurities, such as soluble proteins and metabolites, while preserving extracellular vesicles, the total secretome obtained during and at the end of the 15-day co-culture was subjected to Tangential Flow Filtration (TFF) using the M1 Benchtop TFF system (Bionet). In TFF the sample flows perpendicularly to the filtration direction, preventing filter saturation and enabling continuous processing. For the concentration of the secretome, a 50 kDa hollow fiber membrane (Repligen) was used with the aim of separating the desired components from unwanted smaller impurities.

The TFF system consists of a sterile glass tank that is filled manually through one of the ports on its top cover. A recirculation pump conducts the secretome from the tank to the filter unit; the "retentate" volume is recirculated back into the tank, while the "permeate" is separated from the recirculation loop. The pore-size of the membrane can be adjusted to purify either the total secretome or specifically isolate microvesicles.

In addition to removing the smallest particles, the sample was passively concentrated during continuous circulation through the TFF system, with the retentate volume gradually reduced as permeate was removed. Once the volume reached approximately 1.5 L, a diafiltration process (replacing the culture medium with PBS) was performed by maintaining a constant volume in the tank through the continuous addition of PBS at a rate equal to the filtrate outflow, thereby enabling buffer exchange without further volume reduction.

### 1.6 Viral clearance

In order to eliminate any possible viral particles, the sample, in this case secretome, was subjected to a methylene blue and light treatment. The standard protocol performed was as follows: The sample was mixed with a methylene blue solution (Sigma Aldrich) to achieve a final concentration of 0.3 µg/mL of methylene blue. Next, 150 mL of this mixture was transferred into 1 L square glass bottles (Pyrex), which were placed horizontally in a cabinet under a red LED lamp. The sample was irradiated for 15 minutes with visible light at a wavelength of 630 nm. Following irradiation, the sample was processed through .TFF using a 3 kDa hollow fiber membrane (Repligen), effectively removing methylene blue while preserving the composition of the sample. The virus-free sample was then collected in glass bottles.

### 1.7 Lyophilization

Before lyophilization, 0.01 g of mannitol (Sigma Aldrich) per mL of sample were added in a laminar flow hood, a Grade A or B GMP environment. Mannitol acts as a cryoprotectant to prevent damage during freezing. Previously autoclaved, 5 mL apyrogenic glass vials were filled with 2 mL of this mixture, sealed with sterile rubber stoppers, and stored at -80°C for 48 hours. The vials were then removed from the freezer and placed on the freeze-dryer trays of the Lyovapor L-300 Pro lyophilization system (Buchi), where the following freeze-drying program was performed:
- Primary drying by bringing the vacuum to 10 Pa (0.1 mbar) and the temperature to -40°C;
- Increase heating of the trays by 3°C/hour to -10°C (10 hours), maintaining the vacuum at 10 Pa;
- Lowering vacuum to 5 Pa (0.05 mbar) and maintaining 2 hours at -10°C;
- performing a second heating ramp of 5°C/hour to 25°C (7 hours) and maintain vacuum at 5 Pa;
- Start the secondary drying process keeping the product at 25°C and at maximum vacuum (typically less than 1 Pa (0.01 mbar));
- After 8 hours of drying, perform a vacuum test every hour, when the vacuum loss value is repeated the product is ready;
- The vacuum is broken with nitrogen, the freeze dryer is opened and under laminar flow in environment A or B, all vials are removed and closed with the grinder.

The final product is a dry, powdered secretome concentrate that can be stored in the refrigerator at temperatures between 2 and 8°C, or even frozen at -20°C. For reconstitution, 2 mL of PBS were added to obtain the 1X concentration.

### 1.8. Analysis of the secretome

Cytokine analysis was performed using a commercial kit (R&D Systems). To begin, 50 µL of the sample was transferred into microplate wells, along with magnetic microbeads conjugated to the correspondent antibodies. The mixture was incubated with stirring for 2 hours. After incubation, the microplate was placed over a magnet to attract the microbeads to the bottom of the wells, and the wells were washed to remove unbound components. Next, biotin was added to each well, and the samples were incubated with stirring for 1 hour. The wash step was repeated, and streptavidin was then added. The plate was incubated again with stirring for 30 minutes, followed by a final wash. The samples were subsequently analyzed using a Luminex 200 multiplexing instrument, and the results were processed with the corresponding software.

**Table 1: Cytokines levels (pg/mL) in the secretome and obtained values. LLOQ: below the detection limit. LLOQ-10: below 10 pg/mL, LLOQ-100: below 100 pg/mL.**

| | | | | | | |
|---|---|---|---|---|---|---|
| **TIMP-1** | **IL-1Ra** | **IL-6** | **MMP-1** | **MMP-3** | **IGF-1** | **MIP-1 A** |
| 80000-1000000 | 10000-100000 | <LLOQ-350 | 100-1000 | 10-300 | 10-300 | 1-100 |
| **SDF-1A** | **IL-27** | **LIF** | **IL-1 BETA** | **IL-2** | **IL-4** | **IL-5** |
| 1-100 | <LLOQ | <LLOQ-10 | <LLOQ | <LLOQ | <LLOQ | <LLOQ |
| **IP-10** | IL-7 | IL-8 | IL-10 | PIGF-1 | EOTAXIN | IL-12 p70 |
| <LLOQ-20 | <LLOQ | <LLOQ-100 | <LLOQ-100 | <LLOQ | <LLOQ-10 | <LLOQ |
| **IL-13** | **IL-17A** | **IL-31** | **SCF** | **RANTES** | **IFN-G** | **GM-CSF** |
| <LLOQ | <LLOQ | <LLOQ | <LLOQ-10 | <LLOQ-10 | <LLOQ-3 | <LLOQ |
| **TNF-A** | **HGF** | **MIP-1B** | **IFN-A** | **MCP-1** | **IL-9** | **VEGF-D** |
| <LLOQ-10 | 50-400 | 30-150 | <LLOQ | 100-2000 | <LLOQ | <LLOQ |
| **TNF-B** | **NGF B** | **EGF** | **BDNF** | **GRO -alfa** | **IL-1A** | **IL-23** |
| <LLOQ | <LLOQ | <LLOQ | <LLOQ | LLOQ-10 | <LLOQ | <LLOQ |
| **IL-15** | **IL-18** | **IL-21** | **FGF-2** | **IL-22** | **PDGF-BB** | **VEGF-A** |
| <LLOQ | <LLOQ | <LLOQ | <LLOQ | <LLOQ | <LLOQ-10 | <LLOQ-100 |

### Example 2. Production of exosomes and microvesicles by co-culture of ASCs and M2a macrophages

This example is a variation of Example 1, in which the final product consists of a combination of exosomes and microvesicles. All steps remain the same as in Example 1, except for the TFF step described in section 1.5. For the concentration of exosomes and microvesicles, a 100 - 300 kDa hollow fiber membrane (Repligen) was used, effectively discarding soluble proteins with a molecular weight smaller than 100 - 300 kDa, while maintaining exosomes and microvesicles in the retentate.

### Example 3. Production of exosomes or microvesicles by co-culture with ASCs and M2a macrophages

This example is a variation of Example 1 and Example 2, in which the final product consists of either exosomes or microvesicles, isolated separately. All steps remain the same as in Example 1 and Example 2, except for the TFF step described in section 1.5. After an initial filtration using a 100 kDa hollow fiber membrane, a second filtration was performed using a 0.2 µm hollow fiber membrane (Repligen). This process effectively collects exosomes in the permeate, while retaining larger microvesicles in the retentate. For further isolation of larger microvesicles, a membrane of 0.45 µm pore size (Sartorius) or 0.65 µm pore size (Repligen) was used.

### 3.1. Analysis of the exosomes

Orthogonal methods were employed for exosome analysis. First, a Leprechaun (Unchained Labs) instrument was used to visualize the membrane markers of the obtained exosomes, analyze their distribution, and determine their cargo (Nguyen DDN et al. (2024). *Skin rejuvenation and photoaging protection using adipose-derived stem cell extracellular vesicles loaded with exogenous cargos.* Skin Res Technol. 30(2):e13599).

Following the standard protocol provided by the Leprechun Exosome Human Tetraspanin Kit (Unchained Labs), exosome samples were incubated overnight on chips with immobilized tetraspanin antibodies to facilitate exosome capture. The samples were then washed and incubated for 1 hour with fluorescently labeled antibodies targeting CD9, CD63 and CD81. After a final wash to remove unbound antibodies, the chips were dried and analyzed using the Leprechaun instrument. Complementing this, the size distribution and zeta potential of the exosomes were characterized using Dynamic Light Scattering (DLS) on the Litesizer DLS 700 (Anton Paar) instrument.

Since the biological properties of exosomes are largely influenced by their parent cells, the identity and purity of human Adipose-Derived Mesenchymal Stem Cells (ASCs) were confirmed via flow cytometry using the Cytek Northern Lights spectral cytometer. The analysis evaluated the expression of key mesenchymal markers and the absence of hematopoietic contaminants. This ensures that our exosomes preparations originated from a well-defined and standardized MSC population. For ASC characterization, four positive markers (CD44, CD73, CD90 and CD105) and one negative marker (CD45) were selected. Fluorochromes for each marker were chosen based on their brightness and spectral compatibility. Cells were incubated with a cocktail of fluorochrome-conjugated antibodies (BioLegend), washed with PBS to remove unbound antibodies, and subsequently analyzed by spectral flow cytometer. After gating the ASC population, the spectral signatures were validated: positive markers exhibited strong fluorescence intensity, whereas the negative marker showed nearly undetectable expression.

### 3.2 Quality Control

To ensure the absence of microbial or fungal contamination in the exosome preparation, sterility testing was conducted following the protocol provided by standard sterility testing kits (Applied Biosystems).

Functional assays and/or potency tests were carried out to verify the biological activity of the exosomes, such as their ability to promote MSCs proliferation or induce anti-inflammatory responses.

This protocol ensures the production of high-quality exosomes through the indirect co-culture of ASCs and M2 macrophages encapsulated in a bioreactor, which can be used in regenerative medicine applications, including pancreatic islet regeneration for diabetes treatment.

**Table 2: Potency specification and values for DS batches of exosome**

| | | |
|---|---|---|
| Preliminary Specification: Total potency ≥ 20%) | DS Exosome 20% or more of inflammation decrease in PBMCs cells, with IL-1B. | Batch Exosome |
| | | Batch 2024001 |
| | | Total potency 45,6% |
| | | (Reduction of IL-1β) |
| | | Batch 2024002 |
| | | Total potency 55,8% |
| | | (Reduction of IL-1β) |
| | | Batch 2024003 |
| | | Total potency 49,3% |
| | | (Reduction of IL-1β) |

**Table 3: Sterility specification and values for DS and DP batches of exosome**

| | | |
|---|---|---|
| Preliminary Specification: | DS exosome | DP exosome |
| Sterile | Batch 2024001: Sterile | Batch 2024001A: Sterile |
| | Batch 2024002: Sterile | Batch 2024001 B: Sterile |
| | Batch 2024003: Sterile | Batch 2024002A: Sterile |
| | | Batch 2024002B: Sterile |
| | | Batch 2024003A: Sterile |
| | | Batch 2024003B: Sterile |

**Table 4: Bacterial endotoxins specification and values for DS and DP batches of the exosome.**

| | | |
|---|---|---|
| Preliminary Specification: | DS Exosome | DP Exosome |
| ≤ 5 EU/mL | Batch 2024001: ≤ 0.250 EU/mL | Batch 2024001A: <0.009 EU/mL |
| | Batch 2024002: ≤ 0.250 EU/mL | Batch 2024001 B: <0.01 EU/mL |
| | | Batch 2024002A: <0.04 EU/mL |
| | Batch 2024003: ≤ 0.250 EU/mL | Batch 2024002B: < 0.050 EU/mL |
| | | |
| | | Batch 2024003A: < 0.050 EU/mL |
| | | Batch 2024003B: < 0.065 EU/mL |

**Table 5: pH specification and values for DS and DP batches of the exosome.**

| | | |
|---|---|---|
| Specification: | DS Exosome | DP Exosome |
| 7.5-8.5 (24-25 °C) | Batch 2024001: 7.78 | Batch 2024001A: 7.97 |
| | Batch 2024002: 7.6 | Batch 2024001 B: 7.85 |
| | Batch 2024003: 7.82 | Batch 2024002A: 8.1 |
| | | Batch 2024002B: 8.05 |
| | | Batch 2024003A: 7.95 |
| | | Batch 2024003B: 7.82 |

**Table 6: Residual DNA specification and values for DS and DP batches of the exosome.**

| | | |
|---|---|---|
| Specification: | DS Exosome | DP Exosome |
| <1 ng/mL | Batch 2024001: < 0.5 ng/mL | Batch 2024001A: < 0.5 ng/mL |
| | Batch 2024002: < 0.5 ng/mL | Batch 2024001 B: < 0.5 ng/mL |
| | Batch 2024003: < 0.5 ng/mL | Batch 2024002A: < 0.5 ng/mL |
| | | Batch 2024002B: < 0.5 ng/mL |
| | | Batch 2024003A: < 0.5 ng/mL |
| | | Batch 2024003B: < 0.5 ng/mL |

**Table 7: Total protein content specification and values for DS and DP batches of the exosome.**

| | | |
|---|---|---|
| Specification: | DS Exosome | DP Exosome |
| 300-2,500 µg/mL | Batch 2024001: 1,982.88 µg/mL | Batch 2024001A: 1,594.32 µg/mL |
| | | Batch 2024001B: 1,632.02 µg/mL |
| | Batch 2024002: 2,291.39 µg/mL | Batch 2024002A: 1,545.98 µg/mL |
| | | Batch 2024002B: 1,609.11 µg/mL |
| | Batch 2024003: 1975.11 µg/mL | Batch 2024003A: 1,566.9 µg/mL |
| | | Batch 2024003B: 1,600.33 µg/mL |

**Table 8: Aggregates specification and values for DS and DP batches of the exosome.**

| Specification DS Exosome | Specification DP Exosome |
|---|---|
| Particle size ≤ 90nm. Polydispersion ≤ 30% | Particle size ≤ 90nm. Polydispersion ≤ 30% |
| Batch 2024001: Particle 41.35. Polydisp 24.4% | Batch 2024001A: Particle 40.35. Polydisp 27.4% |
| | Batch 2024001B: Particle 39.35. Polydisp 26.1% |
| Batch 2024002: Particle 39.36. Polydisp 25.8% | Batch 2024002A: Particle 41.15. Polydisp 24.2% |
| | Batch 2024002B: Particle 39.85. Polydisp 26.1% |
| Batch 2024003Particle 40.08. Polydisp 24.2% | Batch 2024003A: Particle 41.01. Polydisp 26.0% |
| | Batch 2024003B: Particle 39.78. Polydisp 23.2% |

**Table 9: Bioburden specification and values for DS and DP batches of the exosome.**

| Specification DS Exosome | Specification DP Exosome |
|---|---|
| TAMC: ≤ 10³ UFC/mL. TYMC: ≤ 10² UFC/mL | TAMC: ≤ 10³ UFC/mL. TYMC: ≤ 10² UFC/mL |
| Batch 2024001: TAMC ≤ 10 UFC/mL. TYMC ≤ 10 UFC/mL | Batch 2024001A: TAMC ≤ 10 UFC/mL. TYMC ≤ 10 UFC/mL |
| Batch 2024002: TAMC ≤ 10 UFC/mL. TYMC ≤ 10 UFC/mL | Batch 2024001B: TAMC ≤ 10 UFC/mL. TYMC ≤ 10 UFC/mL |
| Batch 2024003: TAMC ≤ 10 UFC/mL. TYMC ≤ 10 UFC/mL | Batch 2024002A: < TAMC ≤ 10 UFC/mL. TYMC ≤ 10 UFC/mL |
| | Batch 2024002B: TAMC ≤ 10 UFC/mL. TYMC ≤ 10 UFC/mL |
| | Batch 2024003A: TAMC ≤ 10 UFC/mL. TYMC ≤ 10 UFC/mL |
| | Batch 2024003B: < TAMC ≤ 10 UFC/mL. TYMC ≤ 10 UFC/mL |

**Table 10: Mycoplasma specification and values for DS and DP batches of the exosome.**

| DS Exosome | DP Exosome |
|---|---|
| Specification: Absence/negative | Specification: Absence/negative |
| Batch 2024001: Absence | Batch 2024001A: Absence |
| Batch 2024002: Absence | Batch 2024001 B: Absence |
| Batch 2024003: Absence | Batch 2024002A: Absence |
| | Batch 2024002B: Absence |
| | Batch 2024003A: Absence |
| | Batch 2024003B: Absence |

**Table 11: Endotoxins specification and values for DS and DP batches of the exosome.**

| Specification | Specification |
|---|---|
| ≤ 5 EU/mL | ≤ 5 EU/mL |
| Batch 2024001: 0.021 EU/mL | Batch 2024001A: < 0.005EU/mL |
| Batch 2024002: 0.014EU/mL | Batch 2024001B: < 0.015EU/mL |
| Batch 2024003: 0.004EU/mL | Batch 2024002A: < 0.008EU/mL |
| | Batch 2024002B: < 0.010EU/mL |
| | Batch 2024003A: < 0.006EU/mL |
| | Batch 2024003B: < 0.011EU/mL |

**Table 12: Viral safety specification and values for DS and DP batches of the exosome.**

| Specification DS Exosome | Specification DP Exosome |
|---|---|
| In Vitro adventitious virus determination PCR | In Vitro adventitious virus determination Cytopathic effect in 3 cell lines |
| Specification: Absence/negative | Batch 2024001A: Absence |
| | Batch 2024001 B: Absence |
| Batch 2024001: Absence | Batch 2024002A: Absence |
| Batch 2024002: Absence | Batch 2024002B: Absence |
| Batch 2024003: Absence | Batch 2024003A: Absence |
| | Batch 2024003B: Absence |

**Table 13: Osmolarity specification and values for DS and DP batches of the exosome.**

| Specification DS Exosome | Specification DP Exosome |
|---|---|
| Specification: 200-400 | Specification: 200-400 |
| Batch 2024001: 268.23 | Batch 2024001A: 262.44 |
| Batch 2024002: 257.12 | Batch 2024001B: 271.88 |
| Batch 2024003: 271.91 | Batch 2024002A: 259.24 |
| | Batch 2024002B: 266.29 |
| | Batch 2024003A: 253.71 |
| | Batch 2024003B: 260.01 |

### Example 4. Effect of the exosome in insulin production

For the insulin secretion experiment, a standard model of human pancreatic beta cells was cultured in their specific culture media in coated wells to enhance cell adhesion. All reagents and the protocol followed were provided by Human Cell Design. Cells were treated on day 3 and 5 of culture with IFN-γ (50 ng/mL) and different doses of exosomes (1/4X, 1/2X, 1X, 2X or 4X) or baricitinib (3 mM) (Selleckchem) as a control for IFN-γ inhibition. On day 6, the culture medium was changed for starvation medium and were treated again. After 24 hours, cells were washed and stimulated or not with 11 mM glucose for 40 minutes. Then, the media was harvested and analyzed using a Human Insulin ELISA kit (Invitrogen), and the results were read with the FLUOstar Omega (BMG Labtech) microplate reader at a wavelength of 540 nm. Figure 11 shows the results.

*The following Examples follow the procedures described in Examples 1 and 3, except where specific modifications are indicated.*

### Example 5 for BIOEX002 production

Objective: To produce BIOEX002, a drug based on lyophilized exosomes generated by indirect co-culture in bioreactor of M2a macrophages and Schwann cells, for the treatment of chronic neuropathic pain.
1. Cell preparation:
   - M2a macrophages: Derived from monocytes isolated from buffy coat using Ficoll-Paque density gradient centrifugation, followed by further separation from other PBMCs through adherence. Differentiation into M2a phenotype is performed in CTS AIM-V medium with appropriate cytokine supplementation.
   - Schwann cells: Cultured at a density of 10.000 cells/cm² on poly-L-lysine-coated vessels in high-glucose DMEM medium (Gibco), supplemented with 5% human platelet lysate (Sigma Aldrich), 1% L-glutamine, 1% penicillin-streptomycin, 1% Schwann cell growth supplement (SCGS, ScienCell), and optionally 0.5% amphotericin B.
   Detailed pre-conditioning protocols, including monocyte- and macrophage-specific medium supplements, as well as culture maintenance, incubation conditions, and subculturing for all cell types, are described in Sections 1.1 and 1.2 of Example 1.
2. Encapsulation:
   Cell encapsulation is conducted according to the method described in Section 1.3 of Example 1, using the same materials and encapsulation system.
3. Co-culture in bioreactor:
   Encapsulated M2a macrophages and Schwann cells are introduced into the bioreactor and co-cultured under the conditions described in Section 1.4 of Example 1, maintaining the vesicle ratio (w/v), bioreactor setup, co-culture conditions, and timing of intermediate and final secretome harvests, with the following modification:
   - Culture medium composition: The first 1.2 L added corresponds to Schwann cell culture medium, followed by 0.8 L of macrophage culture medium. This 1.5:1 ratio must be consistently maintained throughout the co-culture, including during intermediate secretome harvests and subsequent addition of fresh medium.
4. TFF filtration:
   To ensure selective isolation of exosomes, the modified protocol introduced in Example 3 is followed:
   - First filtration: A 100 kDa hollow fiber membrane is used to remove small soluble proteins.
   - Second filtration: A 0.2 µm hollow fiber membrane is used, collecting the exosomes in the permeate, while the retentate is discarded.
5. Viral clearance:
   Viral inactivation is performed using methylene blue photochemical treatment, as described in Section 1.6 of Example 1.
6. Lyophilization:
   Exosome concentrate is freeze-dried following the lyophilization steps detailed in Section 1.7 of Example 1, resulting in a stable, dry formulation.

The final product is a lyophilized exosome concentrate ready to be reconstituted in PBS, and that can be stored between 2 and 8°C for short-term use, or at -20°C for long-term storage.

This process complies with GMP standards, ensuring the quality, purity and efficacy of the drug BIOEX002 for clinical use in the treatment of chronic neuropathic pain.

### Example 6 for BIOEX003 production

Objective: To produce BIOEX003, a drug based on lyophilized exosomes generated by indirect co-culture in bioreactor of M2a macrophages and tenocytes, for the treatment of tendinosis.
1: Cell preparation:
   - M2a macrophages: Derived from monocytes isolated from buffy coat using Ficoll-Paque density gradient centrifugation, followed by further separation from other PBMCs through adherence. Differentiation into M2a phenotype is performed in CTS AIM-V medium with appropriate cytokine supplementation.
   - Tenocytes: Cultured at a density of 5000 cells/cm² in high-glucose DMEM medium, supplemented with 5% human platelet lysate, 1% L-glutamine, 1% penicillin-streptomycin, 1% tenocyte growth supplement (TGS, ScienCell), and optionally 0.5% amphotericin B.
   Detailed pre-conditioning protocols, including monocyte- and macrophage-specific medium supplements, as well as culture maintenance, incubation conditions, and subculturing for all cell types, are described in Sections 1.1 and 1.2 of Example 1.
2: Encapsulation:
   Cell encapsulation is conducted according to the method described in Section 1.3 of Example 1, using the same materials and encapsulation system.
3: Co-culture in bioreactor:
   Encapsulated M2a macrophages and tenocytes are introduced into the bioreactor and co-cultured under the conditions described in Section 1.4 of Example 1, maintaining the vesicle ratio (w/v), bioreactor setup, co-culture conditions, and timing of intermediate and final secretome harvests, with the following modification:
   - Culture medium composition: The first 1.2 L added corresponds to tenocyte culture medium, followed by 0.8 L of macrophage culture medium. This 1.5:1 ratio must be consistently maintained throughout the co-culture, including during intermediate secretome harvests and subsequent addition of fresh medium.
4: TFF filtration:
   To ensure selective isolation of exosomes, the modified protocol introduced in Example 3 is followed:
   - First filtration: A 100 kDa hollow fiber membrane is used to remove small soluble proteins.
   - Second filtration: A 0.2 µm hollow fiber membrane is used, collecting exosomes in the permeate, while the retentate is discarded.
5: Viral clearance:
   Viral inactivation is performed using methylene blue photochemical treatment, as described in Section 1.6 of Example 1.
6: Lyophilization:
   Exosome concentrate is freeze-dried following the lyophilization steps detailed in Section 1.7 of Example 1, resulting in a stable, dry formulation.

The final product is a lyophilized exosome concentrate ready to be reconstituted in PBS, and that can be stored between 2 and 8°C for short-term use, or at -20°C for long-term storage.

This process strictly complies with GMP standards, guaranteeing quality, efficacy and safety of the BIOEX003 product, indicated for the treatment of tendinosis.

### Example 7 for BIOEX004 production

Objective: To produce BIOEX004, a drug based on lyophilized exosomes generated by indirect co-culture in bioreactor of M2a macrophages and osteoblasts, for the treatment of rib fractures.
1: Cell preparation
   - M2a macrophages: Derived from monocytes isolated from buffy coat using Ficoll-Paque density gradient centrifugation, followed by further separation from other PBMCs through adherence. Differentiation into M2a phenotype is performed in CTS AIM-V medium with appropriate cytokine supplementation.
   - Osteoblasts: Cultured at a density of 5000 cells/cm² on poly-L-lysine-coated vessels in high-glucose DMEM medium, supplemented with 5% human platelet lysate, 1% L-glutamine, 1% penicillin-streptomycin, 1% osteoblast growth supplement (OGS, ScienCell), and optionally 0.5% amphotericin B.
   Detailed pre-conditioning protocols, including monocyte- and macrophage-specific medium supplements, as well as culture maintenance, incubation conditions, and subculturing for all cell types, are described in Sections 1.1 and 1.2 of Example 1.
2: Encapsulation:
   Cell encapsulation is conducted according to the method described in Section 1.3 of Example 1, using the same materials and encapsulation system.
3: Co-culture in bioreactor:
   Encapsulated M2a macrophages and osteoblasts are introduced into the bioreactor and co-cultured under the conditions described in Section 1.4 of Example 1, maintaining the vesicle ratio (w/v), bioreactor setup, co-culture conditions, and timing of intermediate and final secretome harvests, with the following modification:
   - Culture medium composition: The first 1.2 L added corresponds to osteoblast culture medium, followed by 0.8 L of macrophage culture medium. This 1.5:1 ratio must be consistently maintained throughout the co-culture, including during intermediate secretome harvests and subsequent addition of fresh medium.
4: TFF filtration:
   To ensure selective isolation of exosomes, the modified protocol introduced in Example 3 is followed:
   - First filtration: A 100 kDa hollow fiber membrane is used to remove small soluble proteins. Second filtration: A 0.2 µm hollow fiber membrane is used, collecting exosomes in the permeate, while the retentate is discarded.
5: Viral clearance:
   Viral inactivation is performed using methylene blue photochemical treatment, as described in Section 1.6 of Example 1.
6: Lyophilization:
   Exosome concentrate is freeze-dried following the lyophilization steps detailed in Section 1.7 of Example 1, resulting in a stable, dry formulation.

The final product is a lyophilized exosome concentrate ready to be reconstituted in PBS, and that can be stored between 2 and 8°C for short-term use, or at -20°C for long-term storage.

This process strictly complies with GMP standards, guaranteeing quality, efficacy and safety of the BIOEX004 product, indicated for the treatment of rib fractures.

### Example 8 for BIOEX005 production

Objective: To produce BIOEX005, a drug based on lyophilized exosomes generated by indirect co-culture in bioreactor of M2a macrophages and skeletal muscle cells (myocytes), for the treatment of muscle fibrillary ruptures.
1: Cell preparation:
   - M2a macrophages: Derived from monocytes isolated from buffy coat using Ficoll-Paque density gradient centrifugation, followed by further separation from other PBMCs through adherence. Differentiation into M2a phenotype is performed in CTS AIM-V medium with appropriate cytokine supplementation.
   - Myocytes: Cultured at a density of 10.000 cells/cm² in high-glucose DMEM supplemented with 5% human platelet lysate, 1% L-glutamine, 1% penicillin-streptomycin, 1% skeletal muscle cell growth supplement (skMCGS, ScienCell), and optionally 0.5% amphotericin B.
   Detailed pre-conditioning protocols, including monocyte- and macrophage-specific medium supplements, as well as culture maintenance, incubation conditions, and subculturing for all cell types, are described in Sections 1.1 and 1.2 of Example 1.
2: Encapsulation:
   Cell encapsulation is conducted according to the method described in Section 1.3 of Example 1, using the same materials and encapsulation system.
3: Co-culture in bioreactor:
   Encapsulated M2a macrophages and myocytes are introduced into the bioreactor and co-cultured under the conditions described in Section 1.4 of Example 1, maintaining the vesicle ratio (w/v), bioreactor setup, co-culture conditions, and timing of intermediate and final secretome harvests, with the following modification:
   - Culture medium composition: The first 1.2 L added corresponds to myocyte culture medium, followed by 0.8 L of macrophage culture medium. This 1.5:1 ratio must be consistently maintained throughout the co-culture, including during intermediate secretome harvests and subsequent addition of fresh medium.
4: TFF filtration:
   To ensure selective isolation of exosomes, the modified protocol introduced in Example 3 is followed:
   - First filtration: A 100 kDa hollow fiber membrane is used to remove small soluble proteins.
   - Second filtration: A 0.2 µm hollow fiber membrane is used, collecting exosomes in the permeate, while the retentate is discarded.
5: Viral clearance:
   Viral inactivation is performed using methylene blue photochemical treatment, as described in Section 1.6 of Example 1.
6: Lyophilization:
   Exosome concentrate is freeze-dried following the lyophilization steps detailed in Section 1.7 of Example 1, resulting in a stable, dry formulation.

The final product is a lyophilized exosome concentrate ready to be reconstituted in PBS, and that can be stored between 2 and 8°C for short-term use, or at -20°C for long-term storage.

This process strictly complies with GMP standards, guaranteeing quality, efficacy and safety of the BIOEX005 product, indicated for the treatment of muscle fibrillar ruptures.

### Example 9 for BIOEX006 production

Objective: To produce BIOEX006, a drug based on lyophilized exosomes generated by indirect co-culture in bioreactor of umbilical cord MSCs (UC-MSCs) and joint chondrocytes, for the treatment of osteoarthritis and/or meniscal lesions.
1: Cell preparation:
   - UC-MSCs: Cultured at a density of 5000 cells/cm² on fibronectin- or CELLstart (Gibco)-coated vessels (100 µL/cm² of CELLstart diluted in DPBS at 1:100), in StemPro MSC SFM medium (Gibco), supplemented with 1% L-glutamine, 1% penicillin-streptomycin, and optionally 0.5% amphotericin B.
   - Chondrocytes: Cultured at a density of 5000 cells/cm² in high-glucose DMEM medium, supplemented with 5% human platelet lysate, 1% L-glutamine, 1% penicillin-streptomycin, 1% chondrocyte growth supplement (CGS, ScienCell), and optionally 0.5% amphotericin B.
   Detailed pre-conditioning protocols, including culture maintenance, incubation conditions, and subculturing for all cell types, are described in Section 1.2 of Example 1.
2: Encapsulation:
   Cell encapsulation is conducted according to the method described in Section 1.3 of Example 1, using the same materials and encapsulation system.
3: Co-culture in bioreactor:
   Encapsulated M2a macrophages and chondrocytes are introduced into the bioreactor and co-cultured under the conditions described in Section 1.4 of Example 1, maintaining the vesicle ratio (w/v), bioreactor setup, co-culture conditions, and timing of intermediate and final secretome harvests, with the following modification:
   - Culture medium composition: The first 1.2 L added corresponds to chondrocyte culture medium, followed by 0.8 L of UC-MSC culture medium. This 1.5:1 ratio must be consistently maintained throughout the co-culture, including during intermediate secretome harvests and subsequent addition of fresh medium.
4: TFF filtration:
   To ensure selective isolation of exosomes, the modified protocol introduced in Example 3 is followed:
   - First filtration: A 100 kDa hollow fiber membrane is used to remove small soluble proteins.
   - Second filtration: A 0.2 µm hollow fiber membrane is used, collecting exosomes in the permeate, while the retentate is discarded.
5: Viral clearance:
   Viral inactivation is performed using methylene blue photochemical treatment, as described in Section 1.6 of Example 1.
6. Lyophilization:
   Exosome concentrate is freeze-dried following the lyophilization steps detailed in Section 1.7 of Example 1, resulting in a stable, dry formulation.

The final product is a lyophilized exosome concentrate ready to be reconstituted in PBS, and that can be stored between 2 and 8°C for short-term use, or at -20°C for long-term storage.

This process strictly complies with GMP standards, guaranteeing quality, efficacy and safety of the BIOEX006 product, indicated for the treatment of osteoarthritis and/or meniscus injuries.

### Example 10 for BIOEX007 production

Objective: To produce BIOEX007, a drug based on lyophilized exosomes generated by indirect co-culture in bioreactor of ASCs and breast cancer cells (MCF7, MDA-MB-231), for the treatment of breast cancer and its metastases.
1: Cell preparation:
   - ASCs: Cultured at a density of 5000 cells/cm² on fibronectin-coated vessels in StemPro MSC SFM medium (Gibco), supplemented with 1% L-glutamine, 1% penicillin-streptomycin, and optionally 0.5% amphotericin B.
   - Breast cancer cells (MCF7, MDA-MB-231): Cultured at a density of 30.000 cells/cm² in DMEM/F12, HEPES medium, supplemented with 5% human platelet lysate.
   Detailed pre-conditioning protocols, including culture maintenance, incubation conditions, and subculturing for all cell types, are described in Section 1.2 of Example 1.
2: Encapsulation:
   Cell encapsulation is conducted according to the method described in Section 1.3 of Example 1, using the same materials and encapsulation system.
3: Co-culture in bioreactor
   Encapsulated ASCs and breast cancer cells are introduced into the bioreactor and co-cultured under the conditions described in Section 1.4 of Example 1, maintaining the vesicle ratio (w/v), bioreactor setup, co-culture conditions, and timing of intermediate and final secretome harvests, with the following modification:
   - Culture medium composition: The first 1.2 L added corresponds to ASC culture medium, followed by 0.8 L of breast cancer cell culture medium. This 1.5:1 ratio must be consistently maintained throughout the co-culture, including during intermediate secretome harvests and subsequent addition of fresh medium.
4: **TFF** filtration:
   To ensure selective isolation of exosomes, the modified protocol introduced in Example 3 is followed:
   - First filtration: A 100 kDa hollow fiber membrane is used to remove small soluble proteins.
   - Second filtration: A 0.2 µm hollow fiber membrane is used, collecting exosomes in the permeate, while the retentate is discarded.
5: Viral clearance:
   Viral inactivation is performed using methylene blue photochemical treatment, as described in Section 1.6 of Example 1.
6: Lyophilization:
   Exosome concentrate is freeze-dried following the lyophilization steps detailed in Section 1.7 of Example 1, resulting in a stable, dry formulation.

The final product is a lyophilized exosome concentrate ready to be reconstituted in PBS, and that can be stored between 2 and 8°C for short-term use, or at -20°C for long-term storage.

This process strictly complies with GMP standards, guaranteeing quality, efficacy and safety of the BIOEX007 product, indicated for the treatment of breast cancer and its metastases.

### Example 11 for BIOEX008 production

Objective: To produce BIOEX008, a set of drugs based on lyophilized exosomes generated by dynamic culture in bioreactor of human dendritic cells with vesicles from tumor cells of any lineage. The exosomes generated act as cellular vaccines, strongly activating specific antitumor T lymphocytes and generating a strong specific immune response against cancer.
1: Cell preparation:
   - Dendritic cells: Derived from monocytes isolated from buffy coat using Ficoll-Paque density gradient centrifugation, followed by further separation from other PBMCs through adherence. Monocytes are cultured at a density of 10.000 cells/cm² in differentiation medium, which consists of CTS AIM-V supplemented with 5% human platelet lysate, 1% L-glutamine, 1% penicillin-streptomycin, 100 ng/mL IL-4, 150 ng/mL GM-CSF, and optionally 0.5% amphotericin B. After reaching 90% confluence, typically 5 - 7 days after seeding, the differentiation medium is replaced with maturation medium. The maturation medium, hereinafter referred as dendritic cell culture medium, consists of CTS AIM-V supplemented with 5% human platelet lysate, 1% L-glutamine, 1% penicillin-streptomycin, 5 ng/mL TNF-α (PeproTech), 5 ng/mL IL-1β, 150 ng/mL IL-6 (PeproTech), 1 µg/mL prostaglandin E2 (PGE2, StemCell), and optionally 0.5% amphotericin B.
   - Tumor cell vesicles: Any specific tumor cell line is cultured in high-glucose DMEM medium, supplemented with 5% human platelet lysate, 1% L-glutamine, 1% penicillin-streptomycin, and optionally 0.5% amphotericin B. After the first 24 hours of culture, cellular stress is induced through nutrient deprivation (removal of human platelet lysate and L-glutamine from the medium) or heat shock (culturing at elevated temperatures, around 42°C). These stress conditions are maintained for 48 - 72 hours to promote vesicle release. Vesicles are then isolated by a two-step centrifugation process: the first at low speed (300 x g) to pellet the cells, followed by ultracentrifugation (100.000 x g) of the supernatant to isolate tumor cell-derived vesicles in the pellet.
   Detailed pre-conditioning protocols, including culture maintenance, incubation conditions, and subculturing for all cell types, are described in in Section 1.2 of Example 1.
2: Encapsulation:
   The encapsulation of dendritic cells is conducted according to the method described in Section 1.3 of Example 1, using the same materials and encapsulation system.
3: Dynamic culture in bioreactor:
   Encapsulated dendritic cells are introduced into the bioreactor and cultured under dynamic conditions, maintaining the bioreactor setup, co-culture conditions, and timing of intermediate and final secretome harvests described in Section 1.4 of Example 1, with the following modification:
   - Culture medium composition: The first 1.2 L added corresponds to dendritic cell culture medium, with microcapsules occupying 10% w/v instead of the standard 5%. After the first 24 hours of static culture, the remaining 0.8 L of dendritic cell culture medium is added, supplemented with previously obtained tumor cell-derived vesicles. This adjustment ensured that when dynamic culture began, the concentration of microcapsules is approximately 5% w/v, as intended.
4: TFF filtration:
   To ensure selective isolation of exosomes, the modified protocol introduced in Example 3 is followed:
   - First filtration: A 100 kDa hollow fiber membrane is used to remove small soluble proteins.
   - Second filtration: A 0.2 µm hollow fiber membrane is used, collecting exosomes in the permeate, while the retentate is discarded.
5: Viral clearance:
   Viral inactivation is performed using methylene blue photochemical treatment, as described in Section 1.6 of Example 1.
6: Lyophilization:
   Exosome concentrate is freeze-dried following the lyophilization steps detailed in Section 1.7 of Example 1, resulting in a stable, dry formulation.

The final product is a lyophilized exosome concentrate ready to be reconstituted in PBS, and that can be stored between 2 and 8°C for short-term use, or at -20°C for long-term storage.

This process strictly complies with GMP standards, guaranteeing quality, efficacy and safety of the product BIOEX008x, indicated as a cell vaccine for specific anti-tumour activation.

### Example 12 for BIOEX009 production

Objective: To produce BIOEX009, a drug based on lyophilized exosomes generated by indirect co-culture in bioreactor of bone marrow MSCs (BM-MSCs) and alveolar macrophages previously placed in contact with heat-killed bacteria typical of lung infections causing ARDS, for the treatment of severe pneumonia and/or ARDS.
1: Cell preparation:
   - Alveolar macrophages: Cultured at a density of 100.000 cells/cm² in CTS AIM-V medium with 5% human platelet lysate, 1% L-glutamine, 1% penicillin-streptomycin, 100 µg/mL Infasurf (ONY Biotech), 10 ng/mL GM-CSF (PeproTech), 5 ng/mL TGF-β1 (PeproTech), 5 ng/mL IL-10, and optionally 0.5% amphotericin B. When cells reach around 90% confluence, heat-killed bacteria (e.g., Heat Killed *Streptococcus Pneumoniae,* HKSP, or Heat Killed *Staphylococcus Aureus,* HKSA) (InvivoGen) are added to the medium at a final concentration of 10⁶ - 10⁸ cells/mL. After 24 hours of incubation, the medium is removed, and the cells are washed with PBS before being harvested for encapsulation.
   - BM-MSCs: Cultured at a density of 5000 cells/cm² on fibronectin-coated vessels in StemPro MSC SFM medium (Gibco), supplemented with 1% L-glutamine, 1% penicillin-streptomycin, and optionally 0.5% amphotericin B.
   Detailed pre-conditioning protocols, including culture maintenance, incubation conditions, and subculturing for all cell types, are described in Section 1.2 of Example 1.
2: Encapsulation:
   Cell encapsulation is conducted according to the method described in Section 1.3 of Example 1, using the same materials and encapsulation system.
3: Co-culture in bioreactor:
   Encapsulated alveolar macrophages and BM-MSCs are introduced into the bioreactor and co-cultured under the conditions described in Section 1.4 of Example 1, maintaining the vesicle ratio (w/v), bioreactor setup, co-culture conditions, and timing of intermediate and final secretome harvests, with the following modification:
   - Culture medium composition: The first 1.2 L added corresponds to BM-MSC culture medium, followed by 0.8 L of alveolar macrophage culture medium. This 1.5:1 ratio must be consistently maintained throughout the co-culture, including during intermediate secretome harvests and subsequent addition of fresh medium.
4: TFF filtration:
   To ensure selective isolation of exosomes, the modified protocol introduced in Example 3 is followed:
   - First filtration: A 100 kDa hollow fiber membrane is used to remove small soluble proteins.
   - Second filtration: A 0.2 µm hollow fiber membrane is used, collecting exosomes in the permeate, while the retentate is discarded.
5: Viral clearance:
   Viral inactivation is performed using methylene blue photochemical treatment, as described in Section 1.6 of Example 1.
6: Lyophilization:
   Exosome concentrate is freeze-dried following the lyophilization steps detailed in Section 1.7 of Example 1, resulting in a stable, dry formulation.

The final product is a lyophilized exosome concentrate ready to be reconstituted in PBS, and that can be stored between 2 and 8°C for short-term use, or at -20°C for long-term storage.

This process strictly complies with GMP standards, guaranteeing quality, efficacy and safety of the BIOEX009 product, indicated for the treatment of severe pneumonia and/or ARDS.

### Example 13 for BIOEX010 production

Objective: To produce BIOEX010, a drug based on lyophilized exosomes generated by indirect co-culture in bioreactor of ASCs and M2a macrophages, for the treatment of either refractory intestinal ulcers related or unrelated to inflammatory bowel disease, or lipedema.
1: Cell preparation:
   - ASCs: Cultured at a density of 5000 cells/cm² on fibronectin-coated vessels in StemPro MSC SFM medium (Gibco), supplemented with 1% L-glutamine, 1% penicillin-streptomycin, and optionally 0.5% amphotericin B.
   - M2a macrophages: Derived from monocytes isolated from buffy coat using Ficoll-Paque density gradient centrifugation, followed by further separation from other PBMCs through adherence. Differentiation into M2a phenotype is performed in CTS AIM-V medium with appropriate cytokine supplementation.
   Detailed pre-conditioning protocols, including monocyte- and macrophage-specific medium supplements, as well as culture maintenance, incubation conditions, and subculturing for all cell types, are described in Sections 1.1 and 1.2 of Example 1.
2: Encapsulation:
   Cell encapsulation is conducted according to the method described in Section 1.3 of Example 1, using the same materials and encapsulation system.
3: Co-culture in bioreactor:
   Encapsulated ASCs and M2a macrophages are introduced into the bioreactor and co-cultured under the conditions described in Section 1.4 of Example 1, maintaining the vesicle ratio (w/v), bioreactor setup, co-culture conditions, and timing of intermediate and final secretome harvest.
4: TFF filtration:
   To ensure selective isolation of exosomes, the modified protocol introduced in Example 3 is followed:
   - First filtration: A 100 kDa hollow fiber membrane is used to remove small soluble proteins.
   - Second filtration: A 0.2 µm hollow fiber membrane is used, collecting exosomes in the permeate, while the retentate is discarded.
5: Viral clearance:
   Viral inactivation is performed using methylene blue photochemical treatment, as described in Section 1.6 of Example 1.
6: Lyophilization:
   Exosome concentrate is freeze-dried following the lyophilization steps detailed in Section 1.7 of Example 1, resulting in a stable, dry formulation.

The final product is a lyophilized exosome concentrate ready to be reconstituted in PBS, and that can be stored between 2 and 8°C for short-term use, or at -20°C for long-term storage.

This process strictly complies with GMP standards, guaranteeing the quality, efficacy and safety of the BIOEX010 product, indicated for the treatment of either refractory intestinal ulcers related or unrelated to inflammatory bowel disease, or lipedema.

### Example 14 for BIOEX011 production

Objective: To produce BIOEX011, a drug based on lyophilized exosomes generated by indirect co-culture in bioreactor of UC-MSCs and M2A macrophages, for the treatment of refractory intestinal ulcers, related or not to inflammatory bowel disease.
1: Cell preparation:
   - UC-MSCs: Cultured at a density of 5000 cells/cm² on fibronectin- or CELLstart (Gibco)-coated vessels (100 µL/cm² of CELLstart diluted in DPBS at 1:100), in StemPro MSC SFM medium (Gibco), supplemented with 1% L-glutamine, 1% penicillin-streptomycin, and optionally 0.5% amphotericin B.
   - M2a macrophages: Derived from monocytes isolated from buffy coat using Ficoll-Paque density gradient centrifugation, followed by further separation from other PBMCs through adherence. Differentiation into M2a phenotype is performed in CTS AIM-V medium with appropriate cytokine supplementation.
   Detailed pre-conditioning protocols, including monocyte- and macrophage-specific medium supplements, as well as culture maintenance, incubation conditions, and subculturing for all cell types, are described in Sections 1.1 and 1.2 of Example 1.
2: Encapsulation:
   Cell encapsulation is conducted according to the method described in Section 1.3 of Example 1, using the same materials and encapsulation system.
3: Co-culture in bioreactor:
   Encapsulated M2a macrophages and Schwann cells are introduced into the bioreactor and co-cultured under the conditions described in Section 1.4 of Example 1, maintaining the vesicle ratio (w/v), bioreactor setup, co-culture conditions, and timing of intermediate and final secretome harvests, with the following modification:
   - Culture medium composition: The first 1.2 L added corresponds to UC-MSC culture medium, followed by 0.8 L of macrophage culture medium. This 1.5:1 ratio must be consistently maintained throughout the co-culture, including during intermediate secretome harvests and subsequent addition of fresh medium.
4: TFF filtration:
   To ensure selective isolation of exosomes, the modified protocol introduced in Example 3 is followed:
   - First filtration: A 100 kDa hollow fiber membrane is used to remove small soluble proteins.
   - Second filtration: A 0.2 µm hollow fiber membrane is used, collecting exosomes in the permeate, while the retentate is discarded.
5: Viral clearance:
   Viral inactivation is performed using methylene blue photochemical treatment, as described in Section 1.6 of Example 1.
6: Lyophilization:
   Exosome concentrate is freeze-dried following the lyophilization steps detailed in Section 1.7 of Example 1, resulting in a stable, dry formulation.

The final product is a lyophilized exosome concentrate ready to be reconstituted in PBS, and that can be stored between 2 and 8°C for short-term use, or at -20°C for long-term storage.

This process strictly complies with GMP standards, guaranteeing quality, efficacy and safety of the BIOEX011 product, indicated for the treatment of pancreatic cancer.

### Example 15 for BIOEX012 production

Objective: To produce BIOEX012, a drug based on lyophilized exosomes generated by dynamic culture in bioreactor of UC-MSCs and exosomes from human dendritic cells that have been in previous co-culture with *Propionibacterium acnes,* for the treatment of acne.
1: Heat-killed *Propionibacterium acnes* preparation:
   Due to the unavailability of commercially heat-killed Propionibacterium acnes, the bacteria are cultured from bacteriological loops containing viable microorganisms (Thermo Scientific) onto pre-warmed Brucella agar plate (Sigma Aldrich).
   - Plate culture and CFU count: The plates are incubated under anaerobic conditions at 37°C for 3 to 7 days, until mature colonies are visible. In cases where the Colony-Forming Unit (CFU) count exceeds 300 colonies per plate, serial dilutions (e.g., 10⁻¹, 10⁻², 10⁻³) are performed to accurately quantify the CFUs of *P*. acnes. Plates containing between 30 and 300 colonies are then counted to determine the CFU/mL.
   - Heat-killed treatment: The colonies are harvested using a sterile loop and transferred to a Falcon tube. To remove residual agar, the suspension is centrifuged at 2500 x g for 15 minutes, followed by removal of the supernatant. The bacterial pellet is resuspended in PBS and subjected to heat-killing at 120°C for 20 minutes. To verify the complete inactivation of the bacteria, an aliquot of the heat-treated suspension is plated onto a fresh agar plate and incubated for 5 days to ensure the absence of colony growth.
2: Cell preparation:
   - UC-MSCs: Cultured at a density of 5000 cells/cm² on fibronectin- or CELLstart (Gibco)-coated vessels (100 µL/cm² of CELLstart diluted in DPBS at 1:100), in StemPro MSC SFM medium (Gibco), supplemented with 1% L-glutamine, 1% penicillin-streptomycin, and optionally 0.5% amphotericin B.
   - Dendritic cells: Derived from monocytes isolated from buffy coat using Ficoll-Paque density gradient centrifugation, followed by further separation from other PBMCs through adherence. Monocytes are cultured at a density of 10.000 cells/cm² in differentiation medium, which consists of CTS AIM-V supplemented with 5% human platelet lysate, 1% L-glutamine, 1% penicillin-streptomycin, 100 ng/mL IL-4, 150 ng/mL GM-CSF, and optionally 0.5% amphotericin B. After reaching 90% confluence, typically 5 - 7 days after seeding, the differentiation medium is replaced with maturation medium. The maturation medium, hereinafter referred as dendritic cell culture medium, consists of CTS AIM-V supplemented with 5% human platelet lysate, 1% L-glutamine, 1% penicillin-streptomycin, 5 ng/mL TNF-α, 5 ng/mL IL-1β, 150 ng/mL IL-6, 1 µg/mL prostaglandin E2 (PGE2), and optionally 0.5% amphotericin B. Heat-killed *P. aureus* are added to the medium at a final concentration of 10⁴ to 10⁶ CFU/mL. After 24 hours of incubation, the medium is removed, and the cells are washed with PBS. The conditioned dendritic cell culture is maintained for an additional 48 - 72 hours to promote exosome release. Exosomes are then isolated by a two-step centrifugation process: the first at low speed (300 x g) to pellet the cells, followed by ultracentrifugation (100.000 x g) of the supernatant to isolate exosome-enriched pellet.
   Detailed pre-conditioning protocols, including culture maintenance, incubation conditions, and subculturing for all cell types, are described in Section 1.2 of Example 1.
3: Encapsulation:
   The encapsulation of dendritic cells is conducted according to the method described in Section 1.3 of Example 1, using the same materials and encapsulation system.
4: Dynamic culture in bioreactor:
   Encapsulated UC-MSCs are introduced into the bioreactor and cultured under dynamic conditions, maintaining the bioreactor setup, co-culture conditions, and timing of intermediate and final secretome harvests described in Section 1.4 of Example 1, with the following modification:
   - Culture medium composition: The first 1.2 L added corresponds to UC-MSC culture medium, with microcapsules occupying 10% w/v instead of the standard 5%. After the first 24 hours of static culture, the remaining 0.8 L of UC-MSC culture medium is added, supplemented with previously obtained dendritic cell-derived exosomes. This adjustment ensured that when dynamic culture began, the concentration of microcapsules is approximately 5% w/v, as intended.
5: TFF filtration:
   To ensure selective isolation of exosomes, the modified protocol introduced in Example 3 is followed:
   - First filtration: A 100 kDa hollow fiber membrane is used to remove small soluble proteins.
   - Second filtration: A 0.2 µm hollow fiber membrane is used, collecting exosomes in the permeate, while the retentate is discarded.
6: Viral clearance:
   Viral inactivation is performed using methylene blue photochemical treatment, as described in Section 1.6 of Example 1.
7: Lyophilization:
   Exosome concentrate is freeze-dried following the lyophilization steps detailed in Section 1.7 of Example 1, resulting in a stable, dry formulation.

The final product is a lyophilized exosome concentrate ready to be reconstituted in PBS, and that can be stored between 2 and 8°C for short-term use, or at -20°C for long-term storage.

This process strictly complies with GMP standards, guaranteeing quality, efficacy and safety of the BIOEX012 product, indicated for the treatment of acne.

### Example 16 for BIOEX013 production

Objective: To produce BIOEX013, a drug based on lyophilized exosomes generated by indirect co-culture in bioreactor of UC-MSCs and pulmonary fibroblasts previously stimulated with TGF-β1, for the treatment of idiopathic pulmonary fibrosis.
1: Cell preparation:
   - UC-MSCs: Cultured at a density of 5000 cells/cm² on fibronectin- or CELLstart (Gibco)-coated vessels (100 µL/cm² of CELLstart diluted in DPBS at 1:100), in StemPro MSC SFM medium (Gibco), supplemented with 1% L-glutamine, 1% penicillin-streptomycin, and optionally 0.5% amphotericin B.
   - Pulmonary fibroblasts: Cultured at a density of 5000 cells/cm² on fibronectin-coated vessels in high-glucose DMEM medium, supplemented with 5% human platelet lysate, 1% L-glutamine, 1% penicillin-streptomycin, 1% fibroblast growth supplement (FGS, ScienCell), 10 ng/mL TFG-β1 (to induce a profibrotic phenotype), and optionally 0.5% amphotericin B.
   Detailed pre-conditioning protocols, including culture maintenance, incubation conditions, and subculturing for all cell types, are described in Section 1.2 of Example 1.
2: Encapsulation:
   Cell encapsulation is conducted according to the method described in Section 1.3 of Example 1, using the same materials and encapsulation system.
3: Co-culture in bioreactor:
   Encapsulated UC-MSCs and pulmonary fibroblasts are introduced into the bioreactor and co-cultured under the conditions described in Section 1.4 of Example 1, maintaining the vesicle ratio (w/v), bioreactor setup, co-culture conditions, and timing of intermediate and final secretome harvests, with the following modification:
   - Culture medium composition: The first 1.2 L added corresponds to UC-MSC culture medium, followed by 0.8 L of pulmonary fibroblast culture medium. This 1.5:1 ratio must be consistently maintained throughout the co-culture, including during intermediate secretome harvests and subsequent addition of fresh medium.
4: TFF filtration:
   To ensure selective isolation of exosomes, the modified protocol introduced in Example 3 is followed:
   - First filtration: A 100 kDa hollow fiber membrane is used to remove small soluble proteins.
   - Second filtration: A 0.2 µm hollow fiber membrane is used, collecting exosomes in the permeate, while the retentate is discarded.
5: Viral clearance:
   Viral inactivation is performed using methylene blue photochemical treatment, as described in Section 1.6 of Example 1.
6: Lyophilization:
   Exosome concentrate is freeze-dried following the lyophilization steps detailed in Section 1.7 of Example 1, resulting in a stable, dry formulation.

The final product is a lyophilized exosome concentrate ready to be reconstituted in PBS, and that can be stored between 2 and 8°C for short-term use, or at -20°C for long-term storage.

This process strictly complies with GMP standards, guaranteeing quality, efficacy and safety of the BIOEX013 product, indicated for the treatment of idiopathic pulmonary fibrosis.

### Example 17 for BIOEX014 Production

Objective: To produce BIOEX014, a drug based on lyophilized exosomes generated by indirect co-culture in bioreactor of ASCs, epidermal keratinocytes and dermal fibroblasts, for the treatment of refractory ulcers.
1: Cell preparation:
   - ASCs: Cultured at a density of 5000 cells/cm² on fibronectin-coated vessels in StemPro MSC SFM medium (Gibco), supplemented with 1% L-glutamine, 1% penicillin-streptomycin, and optionally 0.5% amphotericin B.
   - Epidermal keratinocytes: Cultured at a density of 5000 cells/cm² on fibronectin-coated vessels in high-glucose DMEM medium, supplemented with 5% human platelet lysate, 1% L-glutamine, 1% penicillin-streptomycin, 1% keratinocyte growth supplement (KGS, ScienCell), and optionally 0.5% amphotericin B.
   - Dermal fibroblasts: Cultured at a density of 5000 cells/cm² in high-glucose DMEM medium, supplemented with 5% human platelet lysate, 1% L-glutamine, 1% penicillin-streptomycin, 1% fibroblast growth supplement (FGS), and optionally 0.5% amphotericin B.
   Detailed pre-conditioning protocols, including culture maintenance, incubation conditions, and subculturing for all cell types, are described in in Section 1.2 of Example 1.
2: Encapsulation:
   Cell encapsulation is conducted according to the method described in Section 1.3 of Example 1, using the same materials and encapsulation system.
3: Co-culture in bioreactor:
   Encapsulated ASCs, epidermal keratinocytes and dermal fibroblasts are introduced into the bioreactor and co-cultured under the conditions described in Section 1.4 of Example 1, maintaining the vesicle ratio (w/v), bioreactor setup, co-culture conditions, and timing of intermediate and final secretome harvests, with the following modification:
   - Culture medium composition: The first 1.2 L added corresponds to ASCs culture medium, followed by 0.8 L of high-glucose DMEM medium supplemented with specific supplements for both keratinocytes and fibroblasts. This 1.5:1 ratio must be consistently maintained throughout the co-culture, including during intermediate secretome harvests and subsequent addition of fresh medium.
4: TFF filtration:
   To ensure selective isolation of exosomes, the modified protocol introduced in Example 3 is followed:
   - First filtration: A 100 kDa hollow fiber membrane is used to remove small soluble proteins.
   - Second filtration: A 0.2 µm hollow fiber membrane is used, collecting exosomes in the permeate, while the retentate is discarded.
5: Viral clearance:
   Viral inactivation is performed using methylene blue photochemical treatment, as described in Section 1.6 of Example 1.
6: Lyophilization:
   Exosome concentrate is freeze-dried following the lyophilization steps detailed in Section 1.7 of Example 1, resulting in a stable, dry formulation.

The fnal product is a lyophilized exosome concentrate ready to be reconstituted in PBS, and that can be stored between 2 and 8°C for short-term use, or at -20°C for long-term storage.

This process strictly complies with GMP standards, guaranteeing quality, efficacy and safety of the BIOEX014 product, indicated for the treatment of refractory ulcers.

### Example 18 for BIOEX015 production

Objective: To produce BIOEX015, a drug based on lyophilized exosomes generated by indirect co-culture in bioreactor of UC-MSCs and cardiac myocytes cultured under hypoxic stress, for the treatment of acute myocardial infarction.
1: Cell preparation:
   - UC-MSCs: Cultured at a density of 5000 cells/cm² on fibronectin- or CELLstart (Gibco)-coated vessels (100 µL/cm² of CELLstart diluted in DPBS at 1:100), in StemPro MSC SFM medium (Gibco), supplemented with 1% L-glutamine, 1% penicillin-streptomycin, and optionally 0.5% amphotericin B.
   - Cardiac myocytes: Cultured at a density of 5000 cells/cm² in high-glucose DMEM medium, supplemented with 5% human platelet lysate, 1% L-glutamine, 1% penicillin-streptomycin, 1% cardiac myocyte growth supplement (CMGS, ScienCell), and optionally 0.5% amphotericin B. After reaching 90% confluence, typically 5 - 7 days after seeding, the culture is exposed to severe hypoxic conditions (1% O₂) and maintained for an additional 48 hours to induce cellular stress.
   Detailed pre-conditioning protocols, including culture maintenance, incubation conditions, and subculturing for all cell types, are described in Section 1.2 of Example 1.
2: Encapsulation:
   Cell encapsulation is conducted according to the method described in Section 1.3 of Example 1, using the same materials and encapsulation system.
3: Co-culture in bioreactor:
   Encapsulated UC-MSCs and cardiac myocytes are introduced into the bioreactor and co-cultured under the conditions described in Section 1.4 of Example 1, maintaining the vesicle ratio (w/v), bioreactor setup, co-culture conditions, and timing of intermediate and final secretome harvests, with the following modification:
   - Culture medium composition: The first 1.2 L added corresponds to UC-MSC culture medium, followed by 0.8 L of cardiac myocyte culture medium. This 1.5:1 ratio must be consistently maintained throughout the co-culture, including during intermediate secretome harvests and subsequent addition of fresh medium.
4: TFF filtration:
   To ensure selective isolation of exosomes, the modified protocol introduced in Example 3 is followed:
   - First filtration: A 100 kDa hollow fiber membrane is used to remove small soluble proteins.
   - Second filtration: A 0.2 µm hollow fiber membrane is used, collecting exosomes in the permeate, while the retentate is discarded.
5: Viral clearance:
   Viral inactivation is performed using methylene blue photochemical treatment, as described in Section 1.6 of Example 1.
6: Lyophilization:
   Exosome concentrate is freeze-dried following the lyophilization steps detailed in Section 1.7 of Example 1, resulting in a stable, dry formulation.

The final product is a lyophilized exosome concentrate ready to be reconstituted in PBS, and that can be stored between 2 and 8°C for short-term use, or at -20°C for long-term storage.

This process strictly complies with GMP standards, guaranteeing quality, efficacy and safety of the BIOEX015 product, indicated for the treatment of acute myocardial infarction.

### Example 19 for BIOEX016 production

Objective: To produce BIOEX016, a drug based on lyophilized exosomes generated by indirect co-culture in bioreactor of ASCs and keratinocytes inflamed with IL-17 and TNF-α, for the treatment of psoriasis.
1: Cell preparation:
   - ASCs: Cultured at a density of 5000 cells/cm² on fibronectin-coated vessels in StemPro MSC SFM medium (Gibco), supplemented with 1% L-glutamine, 1% penicillin-streptomycin, and optionally 0.5% amphotericin B.
   - Epidermal keratinocytes: Cultured at a density of 5000 cells/cm² on fibronectin-coated vessels in high-glucose DMEM medium, supplemented with 5% human platelet lysate, 1% L-glutamine, 1% penicillin-streptomycin, 1% keratinocyte growth supplement (KGS), and optionally 0.5% amphotericin B. After reaching 90% confluence, typically 5 - 7 days after seeding, epidermal keratinocyte culture medium is further supplemented with 50 ng/mL IL-17 (PeproTech) and 50 ng/mL TNF-α, and culture is maintained for an additional 48 hours to induce psoriatic inflammation.
   Detailed pre-conditioning protocols, including culture maintenance, incubation conditions, and subculturing for all cell types, are described in Section 1.2 of Example 1.
2: Encapsulation:
   Cell encapsulation is conducted according to the method described in Section 1.3 of Example 1, using the same materials and encapsulation system.
3: Co-culture in bioreactor: Encapsulated ASCs and epidermal keratinocytes are introduced into the bioreactor and co-cultured under the conditions described in Section 1.4 of Example 1, maintaining the vesicle ratio (w/v), bioreactor setup, co-culture conditions, and timing of intermediate and final secretome harvests, with the following modification:
   - Culture medium composition: The first 1.2 L added corresponds to ASC culture medium, followed by 0.8 L of epidermal keratinocyte culture medium. This 1.5:1 ratio must be consistently maintained throughout the co-culture, including during intermediate secretome harvests and subsequent addition of fresh medium.
4: TFF filtration:
   To ensure selective isolation of exosomes, the modified protocol introduced in Example 3 is followed:
   - First filtration: A 100 kDa hollow fiber membrane is used to remove small soluble proteins.
   - Second filtration: A 0.2 µm hollow fiber membrane is used, collecting exosomes in the permeate, while the retentate is discarded.
5: Viral clearance:
   Viral inactivation is performed using methylene blue photochemical treatment, as described in Section 1.6 of Example 1.
6: Lyophilization:
   Exosome concentrate is freeze-dried following the lyophilization steps detailed in Section 1.7 of Example 1, resulting in a stable, dry formulation.

The final product is a lyophilized exosome concentrate ready to be reconstituted in PBS, and that can be stored between 2 and 8°C for short-term use, or at -20°C for long-term storage.

This process strictly complies with GMP standards, guaranteeing quality, efficacy and safety of the BIOEX016 product, indicated for the treatment of psoriasis.

## Claims

1. A method of obtention of an immunomodulatory product comprising a secretome, exosomes or microvesicles, the method comprising the following steps:
a) pre-conditioning of primary cells obtained from a subject with a first culture medium;
b) pre-conditioning of Mesenchymal stem cells (MSCs) or M2- or M1-activated macrophages obtained from a subject with a second culture medium;
c) co-culturing the pre-conditioned cells of steps a) and b) in a bioreactor, comprising the steps of:
i. introducing said first culture medium of step a) fresh into a bioreactor;
ii. introducing the pre-conditioned primary cells obtained in step a) into the bioreactor, wherein the amount of primary cells is of maximal 20% w/v of the culture medium added in step i.;
iii. incubating at a temperature of 35 - 40°C, pH between 7.2 - 7.4, during 12 - 36 h.;
iv. introducing said second culture medium of step b) fresh into the bioreactor, wherein the ratio of primary cell culture medium to MSC- or activated macrophage culture medium is between 1:1 and 2:1;
v. introducing the pre-conditioned MSCs or activated macrophages obtained in step b) in the bioreactor, wherein the amount of MSCs or activated macrophages is of maximal 20% w/v of the culture medium added in step iv., to form a co-culture with the primary cells of step ii., in which each cell type is physically separated from other cell type;
vi. incubating the co-culture of step v. under stirring at a temperature of 35 - 40°C and pH between 7.2 - 7.4 under hypoxia, for the obtention of an enriched medium;
d) collecting said enriched medium of step vi;
e) filtering the enriched culture medium obtained in step d) by complex pulsed Tangential Flow Filtration (TFF), for the obtention of the immunomodulatory product.

2. The method of claim 1, comprising forming microcapsules independently encapsulating said primary cells, MSCs and M2- or M1-activated macrophages before incorporating said cells in the co-culture of step c).

3. The method of claim 2, wherein said microcapsules comprise alginate, alginate/polylysine and/or alginate/chitosan polymers

4. The method of any one of claims 1 to 3, wherein said filtering of step e) comprises a 10 - 50 kDa membrane for the obtention of a purified secretome.

5. The method of any one of claims 1 to 4, wherein said filtering of step e) comprises a 100
- 300 kDa membrane for the obtention of combination of exosomes and microvesicles as immunomodulatory product.

6. The method of any one of claims 1 to 4, wherein said filtering of step e) comprises a 100
- 300 kDa membrane followed by filtering with a 0,1 - 0,2 µm pore size membrane for the obtention of exosomes in a permeate and microvesicles in a retentate as separate immunomodulatory products.

7. The method of claim 6, wherein said retentate of microvesicles is further filtrated with a 0,3 - 1 µm pore size membrane for the obtention of large microvesicles.

8. The method of any one of claims 1 to 7, comprising a step f) of viral clearance of the obtained immunomodulatory product.

9. The method of any one of claims 1 to 8, comprising a step g) of filling of unidose packs in sterile apyrogenic containers under sterile conditions.

10. The method of any one of claims 1 to 9, comprising a step h) of lyophilization of the unidose packs of step f).

11. The method of any one of claims 1 to 10, wherein the primary cells are selected from the group consisting of cardiac myocytes, chondrocytes, fibroblasts, keratinocytes, osteoblasts, Schwann cells, skeletal muscle cells and tenocytes.

12. A pharmaceutical composition comprising the secretome, the exosomes, the microvesicles, or the exosomes and the microvesicles, obtained by the method of any one of claims 1 to 11, and a pharmaceutically acceptable excipient.

13. The pharmaceutical composition of claim 12, for use as a medicament.

14. The pharmaceutical composition of claim 12, for use in the treatment of type-1 diabetes.

15. The pharmaceutical composition of claim 12, for use in the treatment of tendinosis.

16. The pharmaceutical composition of claim 12, for use in the treatment of urinary incontinence wherein the primary cells are myocytes; for use in the treatment of chronic neuropathic pain wherein the primary cells are Schwann cells co-cultured with M2 macrophages; for use in the treatment of pseudoarthrosis or rib or vertebral fractures wherein the primary cells are osteoblasts co-cultured with M2 macrophages; for use in the treatment of antibiotic-resistant infections wherein the primary cells are CD8/CD4 lymphocytes and/or M1 macrophages cocultured with bacteria; for use in the treatment lung storm cytokines associated with CAR-T, acute pancreatitis and/or sepsis wherein the primary cells are adult mesenchymal stem cells co-cultured with M2 macrophages; for use in the treatment of Acute Myocardial Infarction (AMI) wherein the primary cells are cardiomyocytes co-cultured with M2 macrophages; for use in the treatment of stroke wherein the primary cells are glial cells co-cultured with M2 macrophage; for use in the treatment of baldness wherein the primary cells are fibroblasts co-cultured with M2 macrophages; for use in the treatment of dermal aging wherein the primary cells are porcine umbilical cord mesenchymal cells co-cultured with M2 macrophages; for use in the treatment of Benign Prostate Hyperplasia (BPH) wherein the primary cells are prostate epithelial and stromal cells co-cultured with M2 macrophages; or for use in the treatment of solid or liquid tumors, wherein the primary cells are tumor cells tumor-specific cocultured with M1 macrophages.
